# EUROPEAN PATENT APPLICATION

(11) **EP 1 598 341 A1**
(43) Date of publication of application: **23.11.2005**
(21) Application number: 04012153.5
(22) Date of filing: 21.05.2004
(51) Int. Cl.: C07D 217/14, C07D 217/16, A61K 31/472, A61P 3/10

(54) **DPP-IV inhibitors**

(71) Applicant: Santhera Pharmaceuticals (Deutschland) Aktiengesellschaft, 69120 Heidelberg (DE)
(72) Inventor: Edwards, Paul John, 69115 Heidelberg (DE)
(74) Representative: Grünecker, Kinkeldey, Stockmair & Schwanhäusser Anwaltssozietät

(57) **Abstract**

The invention relates to compounds of formula (I) 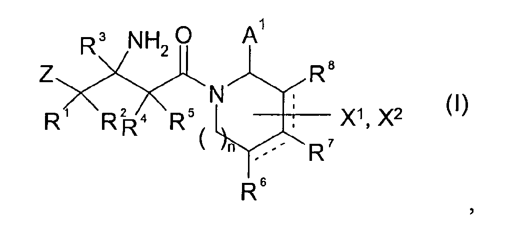 wherein Z, R¹⁻⁸, n, A¹, X¹ and X² have the meaning as cited in the description and the claims. Said compounds are useful as DPP-IV inhibitors. The invention also relates to the preparation of such compounds as well as the production and use thereof as medicament.

## Description

The present invention relates to a novel class of dipeptidyl peptidase inhibitors, including pharmaceutically acceptable salts and prodrugs thereof, which are useful as therapeutic compounds, particularly in the treatment of Type 2 diabetes mellitus, often referred to as non-insulin dependent diabetes mellitus (NIDDM), and of conditions that are often associated with this disease, such as obesity and lipid disorders.

Diabetes refers to a disease process derived from multiple causative factors and characterized by elevated levels of plasma glucose or hyperglycemia in the fasting state or after administration of glucose during an oral glucose tolerance test. Persistent or uncontrolled hyperglycemia is associated with increased and premature morbidity and mortality. Often abnormal glucose homeostasis is associated both directly and indirectly with alterations of the lipid, lipoprotein and apolipoprotein metabolism and other metabolic and hemodynamic disease. Therefore patients with Type 2 diabetes mellitus are at an increased risk of macrovascular and microvascular complications, including coronary heart disease, stroke, peripheral vascular disease, hypertension, nephropathy, neuropathy, and retinopathy. Therefore, therapeutic control of glucose homeostasis, lipid metabolism and hypertension are critically important in the clinical management and treatment of diabetes mellitus.

There are two generally recognized forms of diabetes. In Type 1, or insulin-dependent, diabetes mellitus (IDDM), patients produce little or no insulin, which is the hormone regulating glucose utilization. In Type 2, or noninsulin dependent, diabetes mellitus (NIDDM), patients often have plasma insulin levels that are the same or elevated compared to nondiabetic subjects. These patients develop a resistance to the insulin stimulating effect on glucose and lipid metabolism in the main insulin-sensitive tissues, namely the muscle, liver and adipose tissues. Further, the plasma insulin levels, while elevated, are insufficient to overcome the pronounced insulin resistance.

Insulin resistance is not primarily due to a diminished number of insulin receptors but to a post-insulin receptor binding defect that is not yet understood. This resistance to insulin responsiveness results in insufficient insulin activation of glucose uptake, oxidation and storage in muscle, and inadequate insulin repression of lipolysis in adipose tissue and of glucose production and secretion in the liver.

The available treatments for Type 2 diabetes, which have not changed substantially in many years, have recognized limitations. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Increasing the plasma level of insulin by administration of sulfonylureas (e.g., tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues. However, dangerously low levels of plasma glucose can result from administration of insulin or insulin secretagogues (sulfonylureas or meglitinide), and an increased level of insulin resistance, due to the even higher plasma insulin levels, can occur. The biguanides increase insulin sensitivity resulting in some correction of hyperglycemia. However, the two biguanides, phenformin and metformin, can induce lactic acidosis and nausea/diarrhoea. Metformin has fewer side effects than phenformin and is often prescribed for the treatment of Type 2 diabetes.

The glitazones (*i*.*e*., 5-benzylthiazolidine-2,4-diones) are a recently described class of compounds with potential for ameliorating many symptoms of Type 2 diabetes. These agents substantially increase insulin sensitivity in muscle, liver and adipose tissue in several animal models of Type 2 diabetes, resulting in partial or complete correction of the elevated plasma levels of glucose without occurrence of hypoglycemia. The glitazones that are currently marketed are agonists of the peroxisome proliferator activated receptor (PPAR), primarily the PPAR-gamma subtype. PPAR-gamma agonism is generally believed to be responsible for the improved insulin sensitization that is observed with the glitazones. Newer PPAR agonists that are being tested for treatment of Type 2 diabetes are agonists of the alpha, gamma or delta subtype, or a combination of these, and in many cases are chemically different from the glitazones *(i.e.,* they are not thiazolidinediones). Serious side effects (*e.g*., liver toxicity) have occurred with some of the glitazones, such as troglitazone.

Additional methods of treating the disease are still under investigation. New biochemical approaches that have been recently introduced or are still under development include treatment with alpha-glucosidase inhibitors (*e.g*., acarbose) and protein tyrosine phosphatase-1B (PTP-1B) inhibitors.

Compounds that are inhibitors of the dipeptidyl peptidase-IV (DPP-IV) enzyme are also under investigation as drugs that may be useful in the treatment of diabetes, and particularly Type 2 diabetes. See for example WO-A-97/40832, WO-A-98/19998, WO-A-03/180, WO-A-03/181 and WO-A-2004/007468. The usefulness of DPP-IV inhibitors in the treatment of Type 2 diabetes is based on the fact that DPP-IV *in vivo* readily inactivates glucagon like peptide-1 (GLP-1 ) and gastric inhibitory peptide (GIP). GLP-1 and GIP are incretins and are produced when food is consumed. The incretins stimulate production of insulin. Inhibition of DPP-IV leads to decreased inactivation of the incretins, and this in turn results in increased effectiveness of the incretins in stimulating production of insulin by the pancreas. DPP-IV inhibition therefore results in an increased level of serum insulin. Advantageously, since the incretins are produced by the body only when food is consumed, DPP-IV inhibition is not expected to increase the level of insulin at inappropriate times, such as between meals, which can lead to excessively low blood sugar (hypoglycemia). Inhibition of DPP-IV is therefore expected to increase insulin without increasing the risk of hypoglycemia, which is a dangerous side effect associated with the use of insulin secretagogues.

DPP-IV inhibitors may also have other therapeutic utilities, as discussed elsewhere in this application. DPP-IV inhibitors have not been studied extensively to date, especially for utilities other than diabetes. New compounds are needed so that improved DPP-IV inhibitors can be found for the treatment of diabetes and potentially other diseases and conditions.

Thus, the object of the present invention is to provide a new class of DPP-IV inhibitors which may be effective in the treatment of Type 2 diabetes and other DPP-IV modulated diseases.

Accordingly, the present invention provides novel compounds of formula (I): 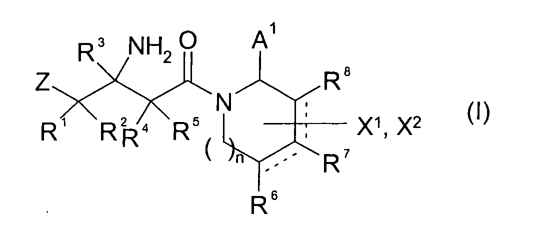 or a pharmaceutically acceptable salt thereof, wherein a dotted line indicates an optionally present double bond and wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R⁹, wherein R⁹ is independently selected from the group consisting of halogen; CN; OH; NH₂; oxo (=O), where the ring is at least partially saturated; R¹⁰; and R¹¹;
R¹⁰ is selected from the group consisting of C₁₋₆ alkyl; O-C₁₋₆ alkyl; and S-C₁₋₆ alkyl, wherein R¹⁰ is optionally interrupted by oxygen and wherein R¹⁰ is optionally substituted with one or more halogen independently selected from the group consisting of F; and Cl;
R¹¹ is selected from the group consisting of phenyl; heterocycle; and C₃₋₇ cycloalkyl, wherein R¹¹ is optionally substituted with one or more R¹², wherein R¹² is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R^{4a};
R², R⁵ are independently selected from the group consisting of H; F; and R^{4b};
R^{4a} is independently selected from the group consisting of C₁₋₆ alkyl; and O-C₁₋₆ alkyl, wherein R^{4a} is optionally substituted with one or more halogen independently selected from the group consisting of F; and Cl;
R^{4b} is C₁₋₆ alkyl, wherein R^{4b} is optionally substituted with one or more halogen independently selected from the group consisting of F; and Cl;
R³ is selected from the group consisting of H; and C₁₋₆ alkyl;
Optionally one or more pairs of R¹, R², R³, R⁴, R⁵ independently selected from the group consisting of R¹/R²; R²/R³; R³/R⁴; and R⁴/R⁵ form a C₃₋₇ cycloalkyl ring, which is optionally substituted with one or more of R¹³, wherein R¹³ is independently selected from the group consisting of F; Cl; and OH;
n is 0, 1 or 2;
X¹ is selected from the group consisting of H; F; Cl; OH; and C₁₋₆ alkyl, optionally substituted with one or more halogen selected from the group consisting of F; and Cl;
X² is selected from the group consisting of H; F; Cl; and C₁₋₆ alkyl, optionally substituted with one or more halogen selected from the group consisting of F; and Cl;
R⁶ and R⁷ form together a ring A, provided that R⁸ is selected from the group consisting of H; F; Cl; Y-H; Y-T; Y¹-H; Y¹-T; and C₁₋₆ alkyl, optionally substituted with one or more halogen selected from the group consisting of F; and Cl;
   or
R⁸ and R⁷ form together a ring A, provided that R⁶ is selected from the group consisting of H; F; Cl; OH; and C₁₋₆ alkyl, optionally substituted with one or more halogen selected from the group consisting of F; and Cl;
A is selected from the group consisting of Z¹; and Z²;
Z¹ is selected from the group consisting of phenyl; naphthyl; and indenyl; wherein Z¹ is optionally substituted with one or more R¹⁴; wherein R¹⁴ is independently selected from the group consisting of halogen; CN; C₁₋₆ alkyl; COOR¹⁵; OR¹⁵; C(O)N(R¹⁵)₂; S(O)₂N(R¹⁵)₂; S(O)N(R¹⁵)₂; N(R¹⁵)S(O)₂R¹⁵; and N(R¹⁵)S(O)R¹⁵;
Z² is selected from the group consisting of C₃₋₇ cycloalkyl; indanyl; tetralinyl; decalinyl; heterocycle; and heterobicycle; wherein Z² is optionally substituted with one or more R¹⁶, wherein R¹⁶ is independently selected from the group consisting of halogen; CN; C₁₋₆ alkyl; OR¹⁷; oxo (=O), where the ring is at least partially saturated; N(R¹⁷)₂; COOR¹⁷; C(O)N(R¹⁷)₂; S(O)₂N(R¹⁷)₂; S(O)N(R¹⁷)₂; N(R¹⁷)S(O)₂R¹⁷; and N(R¹⁷)S(O)R¹⁷;
R¹⁵, R¹⁷ are independently selected from the group consisting of H; C₃₋₇ cycloalkyl; C₁₋₆ alkyl; and -C₁₋₆ alkyl-C₃₋₇ cycloalkyl; wherein each C₃₋₇ cycloalkyl and C₁₋₆ alkyl is optionally substituted with one or more F;
A¹ is selected from the group consisting of F; Cl; and Y-T; provided that when A¹ is Y-T, R⁸ is other than Y-T or Y¹-T;

Optionally A¹ is selected from the group consisting of H; CN; and Y-H, provided that
n is other than 1 or
R⁸ is selected other than to form a ring A; or
R⁸ and R⁷ form a ring A other than pyrimidine;
Y is selected from the group consisting of -C₁₋₆ alkyl-O-T⁰-; -C₁₋₆ alkyl-N(R¹⁸)-T⁰-;
-C₁₋₆ alkyl-S-T⁰- ; -C₁₋₆ alkyl-S(O)-T⁰-; and -C₁₋₆ alkyl-S(O)₂-T⁰-; wherein each C₁₋₆ alkyl is optionally substituted with one or more F;
Y¹ is selected from the group consisting of -O-T⁰-; -N(R^{18a})-T⁰-; -S-T⁰-; -S(O)-T⁰-; and -S(O)₂-T⁰-;
R¹⁸, R^{18a} are independently selected from the group consisting of H; T⁰-H; and T⁰-T;
T⁰ is selected from the group consisting of a covalent bond; -C₁₋₆ alkyl-; -C₁₋₆ alkyl-O-; -C₁₋₆ alkyl-N(R¹⁹)-; -C(O)-; -C(O)-C₁₋₆ alkyl-; -C(O)-C₁₋₆ alkyl-O-; -C(O)-C₁₋₆ alkyl-N(R¹⁹)-; -C(O)O-; -C(O)O-C₁₋₆ alkyl-; -C(O)O-C₁₋₆ alkyl-O-; -C(O)O-C₁₋₆ alkyl-N(R¹⁹)-; -C(O)N(R¹⁹)-; -C(O)N(R¹⁹)-C₁₋₆ alkyl-; -C(O)N(R¹⁹)-C₁₋₆ alkyl-O-; -C(O)N(R¹⁹)-C₁₋₆ alkyl-N(R²⁰)-; -S(O)₂-; -S(O)₂-C₁₋₆ alkyl-; -S(O)₂-C₁₋₆ alkyl-O-; and -S(O)₂-C₁₋₆ alkyl-N(R¹⁹)-; wherein each C₁₋₆ alkyl is optionally substituted with one or more F;
R¹⁹, R²⁰ are independently selected from the group consisting of H; and C₁₋₆ alkyl;
T is selected from the group consisting of T¹; and T²;
T¹ is selected from the group consisting of phenyl; naphthyl; and indenyl; wherein T¹ is optionally substituted with one or more R²¹; wherein R²¹ is independently selected from the group consisting of halogen; CN; R²²; COOH; OH; C(O)NH₂; S(O)₂NH₂; S(O)NH₂; COOT³; OT³; ST³; C(O)N(R²³)T³; S(O)₂N(R²³)T³; S(O)N(R²¹)T³ and T³;
T² is selected from the group consisting of C₃₋₇ cycloalkyl; indanyl; tetralinyl; decalinyl; heterocycle; and heterobicycle; wherein T² is optionally substituted with one or more R²⁴, wherein R²⁴ is independently selected from the group consisting of halogen; CN; R²⁵; OH; oxo (=O), where the ring is at least partially saturated; NH₂; COOH; C(O)NH₂; S(O)₂NH₂; S(O)NH₂; COOT³; OT³; C(O)N(R²⁶)T³; S(O)₂N(R²⁶)T³; S(O)N(R²⁶)T³; N(R²⁶)T³ ; and.T³;
R²² is selected from the group consisting of C₁₋₆ alkyl; O-C₁₋₆ alkyl; S-C₁₋₆ alkyl; COO-C₁₋₆ alkyl; OC(O)- C₁₋₆ alkyl; C(O)N(R²⁷)-C₁₋₆ alkyl; S(O)₂N(R²⁷)-C₁₋₆ alkyl; S(O)N(R²⁷)-C₁₋₆ alkyl; S(O)-C₁₋₆ alkyl; S(O)₂-C₁₋₆ alkyl; N(R²⁷)S(O)₂-C₁₋₆ alkyl; and N(R²⁷)S(O) -C₁₋₆ alkyl; wherein each C₁₋₆ alkyl is optionally substituted with one more R²⁸, wherein R²⁸ is independently selected from the group consisting of F; COOR²⁹; C(O)N(R²⁹R³⁰); S(O)₂N(R²⁹R³⁰); OR²⁹; N(R²⁹R³⁰); T³; O-T³; and N(R²⁹)-T³;
R²⁵ is selected from the group consisting of C₁₋₆ alkyl; O-C₁₋₆ alkyl; S-C₁₋₆ alkyl; N(R³¹)-C₁₋₆ alkyl; COO-C₁₋₆ alkyl; OC(O)- C₁₋₆ alkyl; C(O)N(R³¹)- C₁₋₆ alkyl; N(R³¹)-C(O)-C₁₋₆ alkyl; S(O)₂N(R³¹)-C₁₋₆ alkyl; S(O)N(R³¹)-C₁₋₆ alkyl; S(O)-C₁₋₆ alkyl; S(O)₂-C₁₋₆ alkyl; -N(R³¹)S(O)₂-C₁₋₆ alkyl; and -N(R³¹)S(O)-C₁₋₆ alkyl; wherein each C₁₋₆ alkyl is optionally substituted with one or more R³², wherein R³² is independently selected from the group consisting of F; COOR³³; C(O)N(R³³R³⁴); S(O)₂N(R³³R34_{);} S(O)N(R³³R³⁴); OR³³; N(R³³R³⁴); T³; O-T³; and N(R³³)-T³
R²³, R²⁶, R²⁷, R²⁹, R³⁰, R³¹, R³³, R³⁴ are independently selected from the group consisting of H; and C₁₋₆ alkyl;
T³ is selected from the group consisting of T⁴; and T⁵;
T⁴ is selected from the group consisting of phenyl; naphthyl; and indenyl; wherein T⁴ is optionally substituted with one or more R³⁵, wherein R³⁵ is independently selected from the group consisting of halogen; CN; COOR³⁶; OR³⁶; C(O)N(R³⁶R³⁷); S(O)₂N(R³⁶R³⁷); C₁₋₆ alkyl; O-C₁₋₆ alkyl; S-C₁₋₆ alkyl; COO-C₁₋₆ alkyl; OC(O)-C₁₋₆ alkyl; C(O)N(R³⁶)- C₁₋₆ alkyl; S(O)₂N(R³⁶)-C₁₋₆ alkyl; S(O)N(R³⁶)-C₁₋₆ alkyl; S(O)₂-C₁₋₆ alkyl; S(O) -C₁₋₆ alkyl; N(R³⁶)S(O)₂-C₁₋₆ alkyl; and N(R³⁶)S(O)-C₁₋₆ alkyl; wherein each C₁₋₆ alkyl is optionally substituted with one more halogen selected from the group consisting of F; and Cl;
T⁵ is selected from the group consisting of heterocycle; heterobicycle; C₃₋₇ cycloalkyl; indanyl; tetralinyl; and decalinyl; wherein T⁵ is optionally substituted with one or more R³⁸, wherein R³⁸ is independently selected from the group consisting of halogen; CN; OR³⁹; oxo (=O), where the ring is at least partially saturated; N(R³⁹R⁴⁰); COOR³⁹; C(O)N(R³⁹R⁴⁰); S(O)₂N(R³⁹R⁴⁰); S(O)N(R³⁹R⁴⁰); C₁₋₆ alkyl; O-C₁₋₆ alkyl; S-C₁₋₆ alkyl; N(R³⁹)-C₁₋₆ alkyl; COO-C₁₋₆ alkyl; OC(O)- C₁₋₆ alkyl; C(O)N(R³⁹)- C₁₋₆ alkyl; N(R³⁹)-C(O)-C₁₋₆ alkyl; S(O)₂N(R³⁹)-C₁₋₆ alkyl; S(O)N(R³⁹)-C₁₋ ₆ alkyl; S(O)₂-C₁₋₆ alkyl; S(O)-C₁₋₆ alkyl; N(R³⁹)S(O)₂-C₁₋₆ alkyl; and N(R³⁹)S(O)-C₁₋₆ alkyl; wherein each C₁₋₆ alkyl is optionally substituted with one more halogen selected from the group consisting of F; and Cl;
R³⁶,R³⁷, R³⁹, R⁴⁰, are independently selected from the group consisting of H; and C₁₋₆ alkyl.

Within the meaning of the present invention the terms are used as follows:

In case a variable or substituent can be selected from a group of different variants and such variable or substituent occurs more than once the respective variants can be the same or different.

"Alkyl" means a straight-chain or branched carbon chain that may contain double or triple bonds. It is generally preferred that alkyl doesn't contain double or triple bonds. "C₁₋₄ Alkyl" means an alkyl chain having 1 - 4 carbon atoms, e.g. at the end of a molecule methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl or amid, e.g. -CH₂-, -CH₂-CH₂-, -CH=CH-, -CH(CH₃)-, -C(CH₂)-, -CH₂-CH₂-CH₂-, - CH(C₂H₅)-, -CH(CH₃)₂-. "C₁₋₆ Alkyl" means an alkyl chain having 1 - 6 carbon atoms, e.g. C₁₋₄ alkyl, methyl, ethyl, -CH=CH₂, -C2≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amid, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-. Each hydrogen of a C₁₋₆ alkyl carbon may be replaced by a substituent.

"C₃₋₇ Cycloalkyl" or "C₃₋₇ Cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine.

"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

A preferred stereochemistry of compounds or a pharmaceutically acceptable salt thereof according to the present invention is shown in formula (Ia) 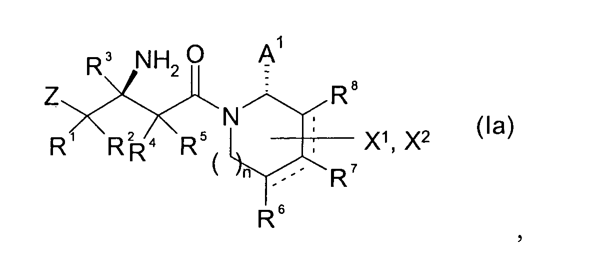 wherein Z, R¹⁻⁸, n, A¹, X¹ and X² have the meaning as indicated above.

Preferred compounds of formula (I) or (Ia) are those compounds in which one or more of the residues contained therein have the meanings given below, with all combinations of preferred substituent definitions being a subject of the present invention. With respect to all preferred compounds of the formulas (I) or (Ia) the present invention also includes all tautomeric and stereoisomeric forms and mixtures thereof in all ratios, and their pharmaceutically acceptable salts.

In preferred embodiments of the present invention, the substituents Z, R¹⁻⁸, n, A¹, X¹ and X² of the formula (I) or (Ia) independently have the following meaning. Hence, one or more of the substituents Z, R¹⁻⁸, n, A¹, X¹ and X² can have the preferred or more preferred meanings given below.

Preferably, Z is selected from the group consisting of phenyl; and heterocycle; wherein Z is optionally substituted with up to 2 R⁹, which are the same or different.

Preferably, R⁹ is selected from the group consisting of F; Cl; CN; and C₁₋₆ alkyl.

Preferably, R¹, R², R⁴, R⁵ are independently selected from the group consisting of H; F; and C₁₋₆ alkyl, optionally substituted with one or more F.

Preferably, R³ is H.

Preferably, n is 0 or 1.

Preferably, X¹, X² are independently selected from the group consisting of H; and F.

Preferably, R⁶ and R⁷ form together a ring A and R⁸ is selected from the group consisting ofH; and F.

Preferably, A is selected from the group consisting of phenyl, optionally substituted with up to 3 R¹⁴, which are the same or different; and C₃₋₇ cycloalkyl, optionally substituted with up to 3 R¹⁶, which are the same or different.

Preferably, R¹⁴, R¹⁶ are independently selected from the group consisting of halogen; CN; and CH₃.

Preferably, A¹ is selected from the group consisting of H; and Y-H and R⁸ is selected other than to form a ring A; and Y is selected from the group consisting of C₁₋₆ alkyl-N(R¹⁸); C₁₋₆ alkyl-N(R¹⁸)-C₁₋₆ alkyl; C₁₋₆ alkyl -N(R¹⁸)-C(O)- C₁₋₆ alkyl; C₁₋₆ alkyl-N(R¹⁸)-C(O)-C₁₋₆ alkyl-O; C₁₋₆ alkyl-N(R¹⁸)-S(O)₂-C₁₋₆ alkyl; and C₁₋₆ alkyl-O, wherein R¹⁸ is selected from the group consisting of H; and C₁₋₆ alkyl. Y is more preferred selected from the group consisting of -CH₂-N(R¹⁸); -CH₂-N(R¹⁸)-CH₂-; -CH₂-N(R¹⁸)-C(O)-CH₂-; -CH₂-N(R¹⁸)-S(O)₂-CH₂-; -CH₂- N(R¹⁸)-C(O)-CH₂-O-; and -CH₂-O-; wherein R¹⁸ is selected from the group consisting of H; and CH₃.

In an also preferred embodiment A¹ is Y-T, wherein Y is selected from the group consisting of C₁₋₆ alkyl-N(R¹⁸); C₁₋₆ alkyl-N(R¹⁸)-C₁₋₆ alkyl; C₁₋₆ alkyl-N(R¹⁸)-C(O); C₁₋₆ alkyl-N(R¹⁸)-C(O)-C₁₋₆ alkyl; C₁₋₆ alkyl-N(R¹⁸)-S(O)₂; C₁₋₆ alkyl-N(R¹⁸)-S(O)₂-C₁₋₆ alkyl; C₁₋₆ alkyl-O; and C₁₋₆ alkyl-O-C₁₋₆ alkyl; and wherein R¹⁸ is selected from the group consisting of H; and C₁₋₆ alkyl. Y is more preferred selected from the group consisting of CH₂-NH-C(O); CH₂-O; CH₂-O-CH₂-; and CH₂-NHS(O)₂.

Preferably, T is T¹ and T¹ is phenyl, optionally substituted with up to 3 R²¹, which are the same or different.

Preferably, R²¹ is F.

In an also preferred embodiment T is T² and T² is selected from the group consisting of C₃₋₇ cycloalkyl; and heterocycle, wherein T² is optionally substituted with up to 3 R²⁴, which are the same or different.

T² is more preferred selected from the group consisting of cyclopropyl; 1,2,4-triazole; and 1,2-oxazole.

Preferably, R²⁴ is selected from the group consisting of OH; NH₂; and CH₃.

Compounds of the formula (I) or (Ia) in which some or all of the above-mentioned groups have the preferred or more preferred meanings are also an object of the present invention.

Preferred embodiments of the compounds according to present invention are:

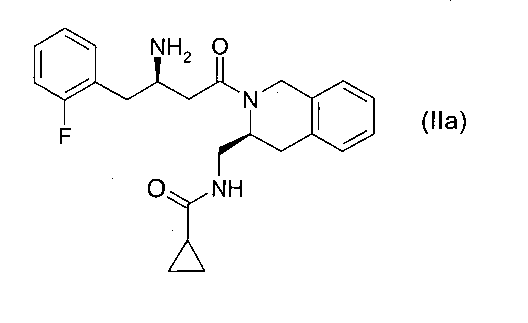

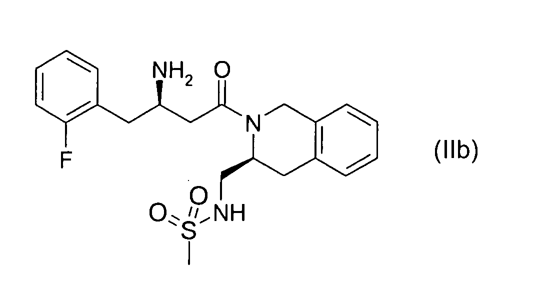

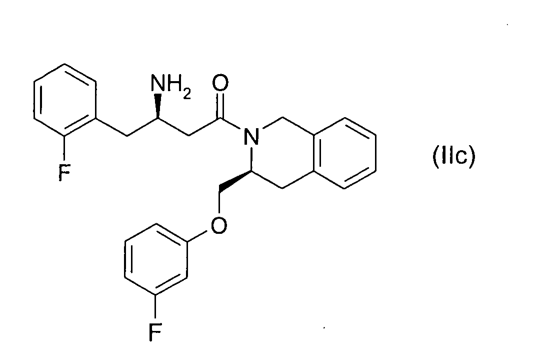

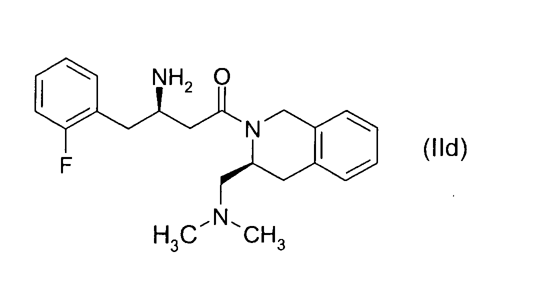

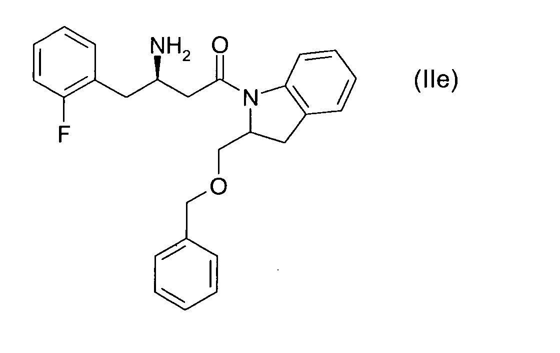

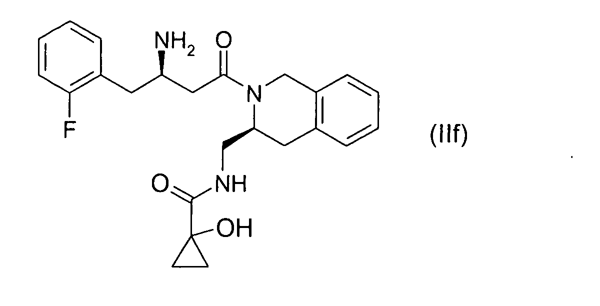

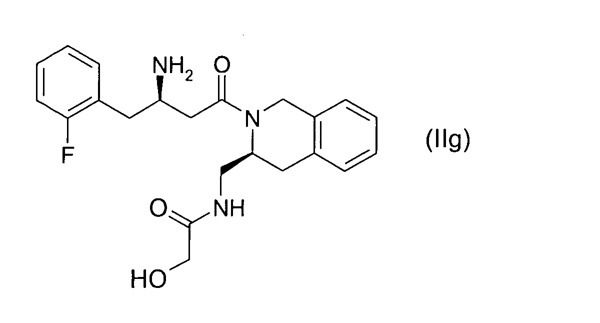

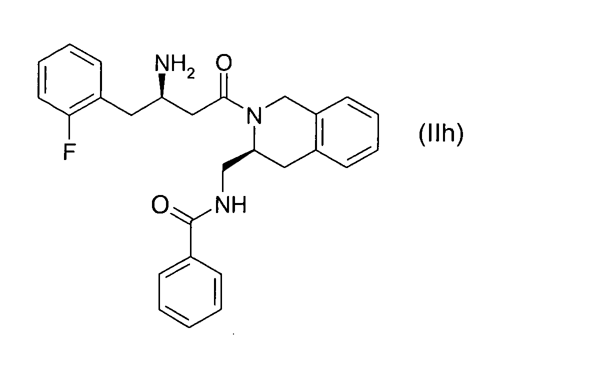

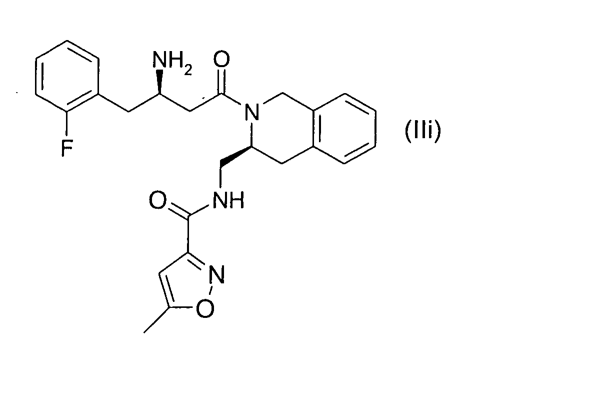

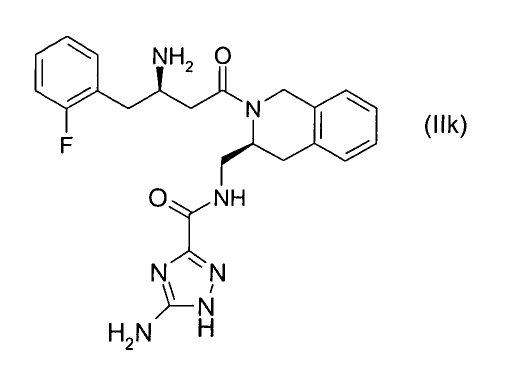

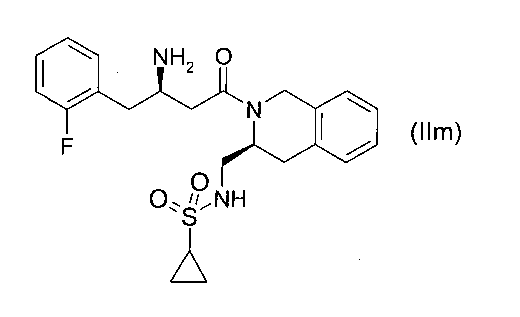

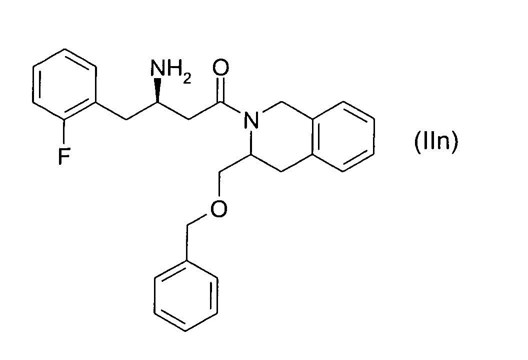

Furthermore, the present invention provides prodrug compounds of the compounds of the invention as described above.
"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I) or (Ia) are also within the scope of the present invention.

Where tautomerism, like e.g. keto-enol tautomerism, of compounds of general formula (I) or (Ia) or their prodrugs may occur, the individual forms, like e.g. the keto and enol form, are claimed separately and together as mixtures in any ratio. Same applies for stereoisomers, like e.g. enantiomers, cis/trans isomers, conformers and the like. If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) or (Ia) may be obtained from stereoselective synthesis using optically pure starting materials.

In case the compounds according to formula (I) or (Ia) contain one or more acidic or basic groups, the invention also comprises their corresponding pharmaceutically or toxicologically acceptable salts, in particular their pharmaceutically utilizable salts. Thus, the compounds of the formula (I) or (Ia) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) or (Ia) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) or (Ia) simultaneously contain acidic and basic groups in the molecule, the invention also includes, in addition to the salt forms mentioned, inner salts or betaines (zwitterions). The respective salts according to the formula (I) or (Ia) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) or (Ia) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or (Ia) or their prodrugs as DPP-IV inhibitors. DPP-IV is a cell surface protein that has been implicated in a wide range of biological functions. It has a broad tissue distribution (intestine, kidney, liver, pancreas, placenta, thymus, spleen, epithelial cells, vascular endothelium, lymphoid and myeloid cells, serum), and distinct tissue and cell-type expression levels. DPP-IV is identical to the T cell activation marker CD26, and it can cleave a number of immunoregulatory, endocrine, and neurological peptides *in vitro.* This has suggested a potential role for this peptidase in a variety of disease processes.

DPP-IV related diseases are described in more detail in WO-A-03/181 under the paragraph "Utilities" which is herewith incorporated by reference.

Accordingly, the present invention provides compounds of formula (I) or (Ia) or their prodrugs or pharmaceutically acceptable salt thereof for use as a medicament.

Furthermore, the present invention provides the use of compounds of formula (I) or (Ia) or their prodrugs or a pharmaceutically acceptable salt thereof for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency. Preferred is non-insulin dependent (Type II) diabetes mellitus and obesity.

The present invention provides pharmaceutical compositions comprising a compound of formula (I) or (Ia), or a prodrug compound thereof, or a pharmaceutically acceptable salt thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like one or more additional compounds of formula (I) or (Ia), or a prodrug compound or other DPP-IV inhibitors.
Other active ingredients are disclosed in WO-A-03/181 under the paragraph "Combination Therapy" which is herewith incorporated by reference. Accordingly, other active ingredients may be insulin sensitizers; PPAR agonists; biguanides; protein tyrosinephosphatase-1B (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoIy dual agonists; inhibitors of cholesterol absorption; acyl CoA : cholesterol acyltransferase inhibitors; anti-oxidants; PPARo agonists; antiobesity compounds; an ileal bile acid transporter inhibitor; or anti-inflammatory agents or pharmaceutically acceptable salts of these active compounds.

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) or (Ia) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) or (Ia) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils. Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) or (Ia) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligrams to about 50 milligrams. In the case of a 70 kg adult human, the total daily dose will generally be from about 7 milligrams to about 350 milligrams. This dosage regimen may be adjusted to provide the optimal therapeutic response.

Preferred embodiments of compounds having formula (I) of the present invention can be prepared from beta amino acid intermediates such as those of formula (II), using standard peptide coupling conditions. The preparation of these intermediates is described in the following schemes.

Some abbreviations that may appear in this application are as follows.

### ABBREVIATIONS

### Designation

- bs: Broad singlet
- Boc (or BOC): *tert-*Butoxycarbonyl
- CDI: *N,N* Carbonyldiimidazole
- DCM: Dichloromethane
- DEAD: Diethyl azodicarboxylate
- DIEA: Diisopropylethylamine
- DMF: N,N-Dimethylformamide
- DPPA: Diphenylphosphorylazide
- EDC: 1-Ethyl-3-(3-dimethylaminopropyl)carbodiimide hydrochloride
- Et₃N: Triethylamine
- Fmoc: 9-Fluorenylmethoxycarbonyl
- HATU: O-(7-Azabenzotriazol-1-yl)-*N*,*N*,*N*', *N*'-tetramethyluronium hexafluorophosphate
- HOBt: 1-Hydroxybenzotriazole
- HPLC: High pressure liquid chromatography
- Pd-C: Palladium on charcoal
- PG: Protecting group
- rt: Retention time
- TEA: triethylamine
- TFA: Trifluoroacetic acid

Available starting materials may be amines or alcohols having the formula (II)

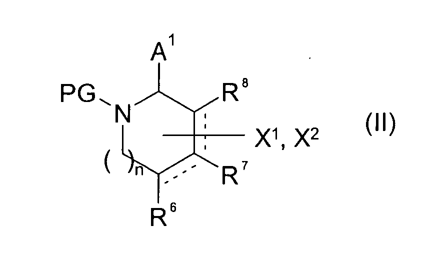

They may be purchased from commercially available sources such as Array, Sigma-Aldrich, Fluka, ABCR or be synthesized by one skilled in the art. Common reactions between compounds containing amino groups and carboxyl, sulfonyl or isocyanate functionalities may be employed for their synthesis with suitable functionalized starting materials. Nucleophilic substitution reactions between compounds containing a suitable leaving group (e.g., halogenide, mesylate, tosylate) and nucleophiles (e.g., amines) may be also employed. The conversion of diverse functional groups (such as esters, alcohols, amides, nitriles, azides) may allow the synthesis of some intermediates or final compounds.

Schemes A through D outline general procedures for the synthesis of some compounds described below. Unless otherwise indicated in the schemes, the variables have the same meaning as described above.

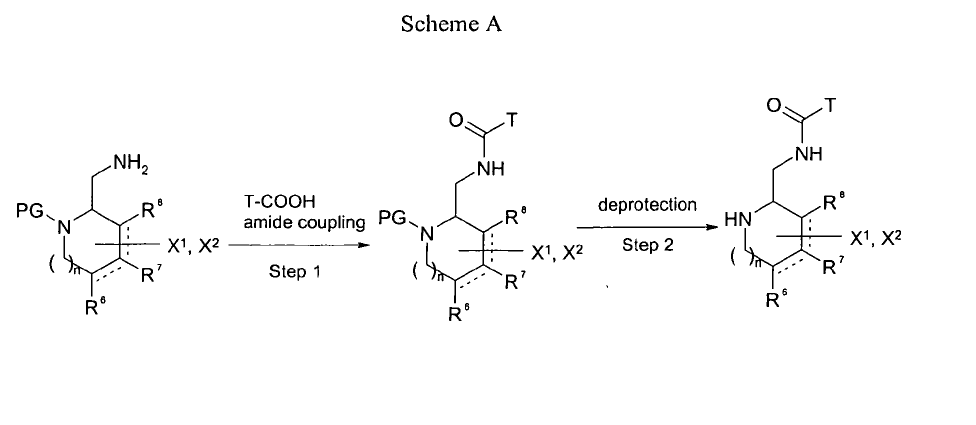

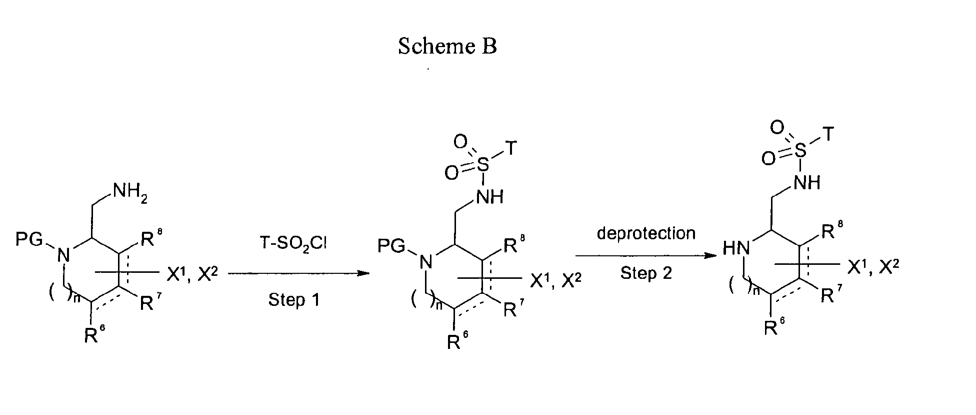

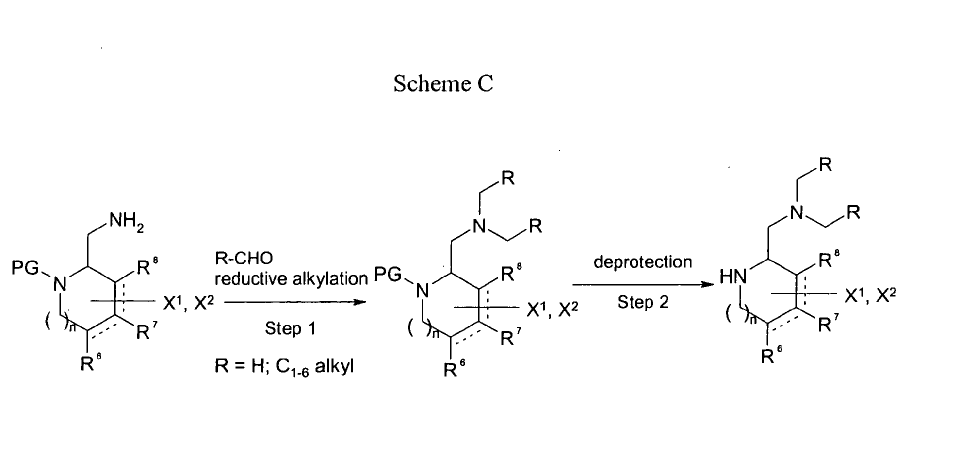

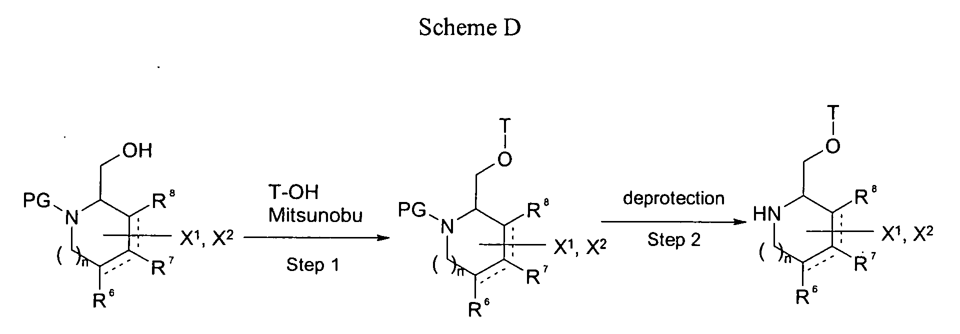

Enantiomerically pure beta amino acids having the formula (III) 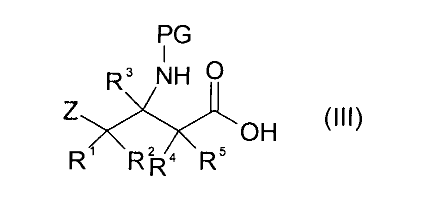 where R¹ - R⁵ is H may be commercially available, known in the literature or may be conveniently synthesised using one of the methods already published and reviewed in e.g., Cole, *Tetrahedron,* 32, 9517 (1994), Juaristi et al., *Aldrichimica Acta,* 27, 3, 1994, or Juaristi, *Enantioselective Synthesis of β-Amino Acids,* Ed. Wiley-VCH, New York, 1997.

Unless otherwise noted, all non-aqueous reactions were carried out under argon atmosphere with commercial dry solvents. Compounds were purified using flash column chromatography using Merck silica gel 60 (230-400 mesh) or reverse phase preparative HPLC using a Reprosil-Pur ODS3, 5 µm, 20 × 125 mm column with Shimadzu LC8A-Pump and SPD-10Avp UV/Vis diode array detector. The ¹H-NMR spectra were recorded on a Varian VXR-S (300 MHz for ¹H-NMR) using d₆-dimethylsulfoxide as solvent; chemical shifts are reported in ppm relative to tetramethylsilane. Analytical LC/MS was performed using Reprosil-Pur ODS3, 5 µM, 1 x 60 mm columns with a linear gradient from 5% to 95% acetonitrile in water (0.1 % TFA) at a flow rate of 250 µl/min; retention times are given in minutes. Methods are: (I) runs on a LC10Advp-Pump (Shimadzu) with SPD-M10Avp UV/Vis diode array detector and QP2010 MS-detector in ESI+ modus with UV-detection at 214, 254 and 275 nm, 10 min. linear gradient; (II) idem but 5 min. linear gradient; (III) runs on a LC10Advp-Pump (Shimadzu) with SPD-10Avp dual wavelength UV-detector and QP2010 MS-detector in ESI+ modus with UV-detection at 214 and 254 nm, 10 min. linear gradient; (IV) idem but 5 min. linear gradient.

### General procedure for making compounds of the invention

In general, compounds having the structure (I) 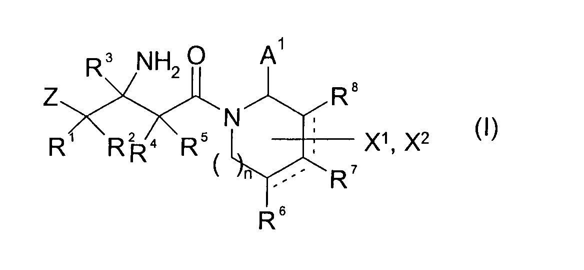 wherein the variables have the above described meanings, may be prepared using standard peptide coupling conditions. For example, it may be possible to use 1-ethyl-3-(3-dimethylaminopropyl)carbodiimide hydrochloride (EDC) in combination with 1-hydroxybenzotriazole (HOBt) and a base (triethylamine or diisopropylethylamine) or O-(7-azabenzotriazol-1-yl)-*N*,*N*,*N*',*N*'-tetramethyluronium hexafluorophosphate (HATU) in the presence of a base, in solvents such as methylene chloride or *N,N* dimethylformamide.

Scheme E outlines a procedure for using the amines formed according to Schemes A through D to synthesize compounds that are embodiments of the invention.

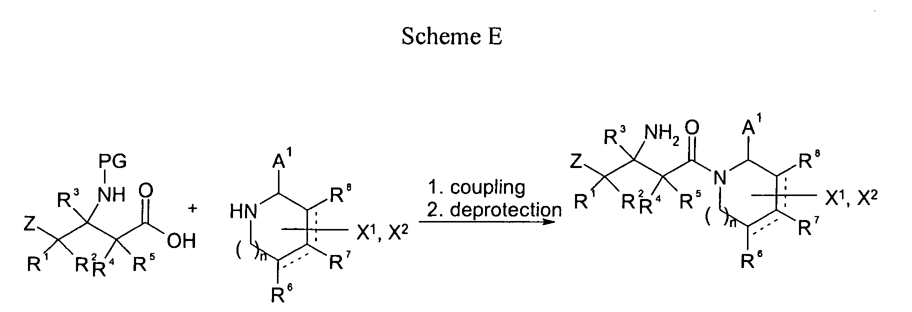

The protective group may be removed with, for example, diethylamine in dichloromethane in the case of 9-fluorenylmethoxycarbonyl or using acidic conditions (such as trifluoroacetic acid in dichloromethane or hydrochloric acid in dioxane) in the case of *tert*-butoxycarbonyl, as described in *Protective Groups in Organic Synthesis* 3^{rd} ed., Ed. Wiley-VCH, New York; 1999.

For the purification of intermediates or end products, flash chromatography on silica gel may be suitable for the free amines whereas the use of preparative HPLC leads to the isolation of the corresponding trifluoroacetic acid salts.

Compounds may be prepared by other means however, and the suggested starting materials and procedures described below are exemplary only and should not be considered as limiting the scope of the invention.

### EXAMPLES

The following examples are provided so that the invention might be more fully understood. These examples are illustrative only and should not be construed as limiting the invention in any way.

### PREPARATIONS

### Example 1

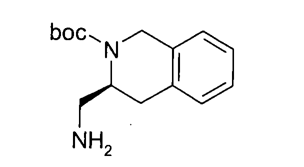

Step 1

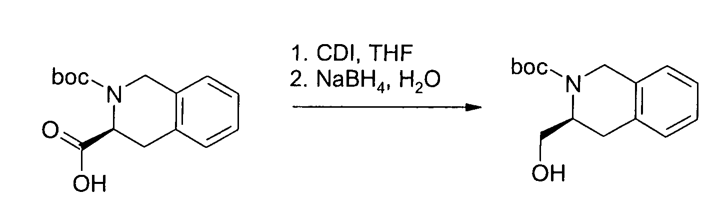

### (3S)3-Hydroxymethyl-3,4-dihydro-1H-isoquinoline-2-carboxylic acid tert-butyl ester

437 mg (1.58 mmol) of (2S)-3,4-Dihydro-1H-isoquinoline-2,3-dicarboxylic acid 2-tert-butyl ester are dissolved in 20 ml dry tetrahydrofuran, then 384 mg (2.37 mmol) N,N'-carbonyldiimidazole added and the mixture stirred at ambient temperature for 30 min. The flask is transferred into an ice bath and 90 mg (2.37 mmol) of sodiumborohydride in 1 ml water added. After stirring for 10 min at 0°C, acetone is added and the solvent removed under reduced pressure. The solid residue is taken into water/ ethylacetate and the layers are separated. The organic layer is washed sequentially with 5% citric acid, saturated sodium bicarbonate solution and brine and dried over sodium sulfate. The solvent is removed and the residue purified by flash chromatography (silica gel, cyclohexane/ethyl acetate 2: 1) to yield the title compound.
LCMS (II) rt 2.62, m/z 164 (M+H-Boc)⁺.
¹H-NMR (300 MHz, DMSO-d₆) δ = 1.43 (s, 9H), 2.85 (m, 2H), 3.13 (m, 1H), 3.30 (m, 1H), 4.13 - 4.30 (m, 2H), 4.60 (d, J = 16.6 Hz, 1H), 4.68 (bs, 1H), 7.13 (s, 4H).

### Step 2

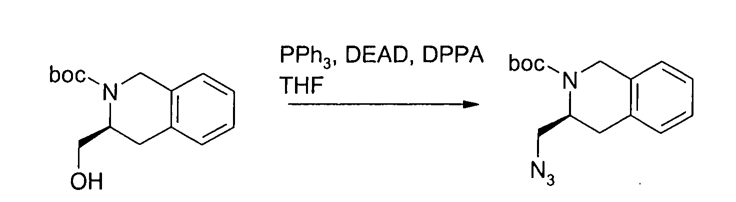

### (3S)-3-Azidomethyl-3,4-dihydro-1H-isoquinoline-2-carboxylic acid tert-butyl ester

Following the procedure from Page et al., J. *Med. Chem.* 44, 2387 (2001) the alcohol from step 1 was transformed into the title compound.
LCMS (II) rt 3.35, m/z 230 (M+H-Boc+CH₃CN)⁺.

### Step 3

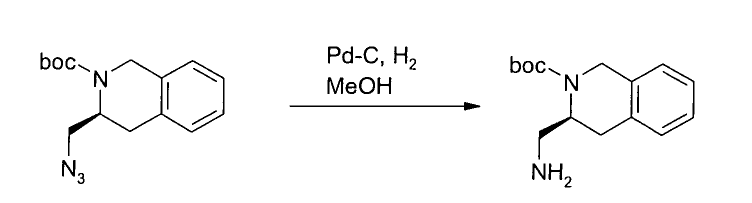

### (3S)-3-Aminomethyl-3,4-dihydro-1H-isoquinoline-2-carboxylic acid tert-butyl ester

6.02 mg (2.09 mmol) of the azide from step 2 are dissolved in 20 ml methanol, 10% Pd-C is added and the reaction stirred for 1.5 h under hydrogen atmosphere. The mixture is filtered over Celite and the solvent removed under reduced pressure. Flash chromatography (silica gel, dichloromethane/methanol with 3% ammonia) afforded of the title compound.
LCMS (II) rt 2.21, m/z 263 (M+H)⁺.
¹H-NMR (300 MHz, DMSO-d₆) δ = 1.43 (s, 9H), 2.37 (dd, J = 12.5 Hz, J = 7.6 Hz, 1H), 2.48 (m together with DMSO, 1H), 2.84 (m, 2H), 4.13 - 4.20 (m, 2H), 4.63 (d, J = 16.7 Hz, 1H), 7.11 (s, 4H).

### Example 2

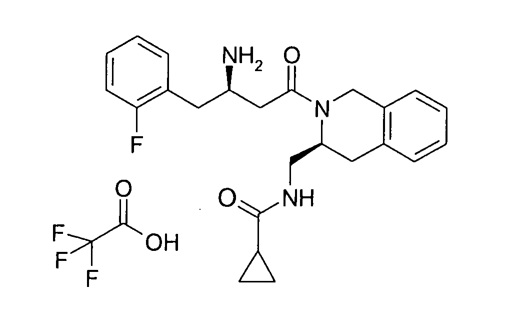

Step 1

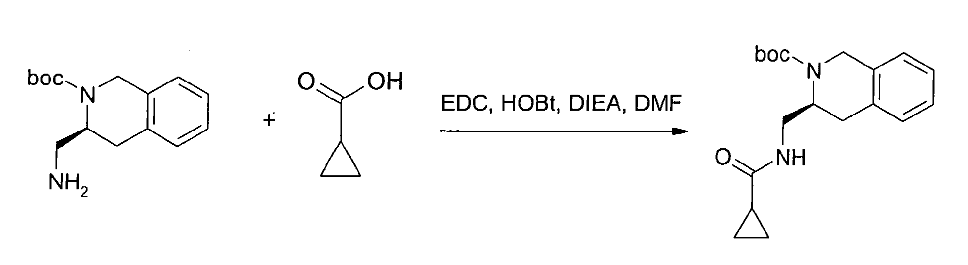

### (3S)-3-[(Cyclopropanecarbonyl-amino)-methyl]-3,4-dihydro-1H-isoquinoline-2-carboxylic acid tert-butyl ester

A mixture of 12 mg (0.14 mmol) cyclopropanecarboxylic acid, 31 mg (0.16 mmol) 1-ethyl-3-(3-dimethylaminopropyl)-carbodiimide hydrochloride (EDC), 22 mg (0.16 mmol) 1-hydroxybenzotriazole (HOBt) and 66 µl (0.35 mmol) diisopropylethylamine (DIEA) in 2 ml dimethylformamide is stirred at ambient temperature for 10 min before 38 mg (0.14 mmol) (3S)-3-Aminomethyl-3,4-dihydro-1H-isoquinoline-2-carboxylic acid tert-butyl ester (example 1) in 1 ml dimethylformamide are added and stirring continued overnight. The solution is diluted with 50 ml ethyl acetate, washed sequentially with 5 % citric acid, saturated aqueous sodium bicarbonate solution, and brine and dried over sodium sulfate. The solvent is removed in vacuo and the residue purified by flash chromatography (silica gel, cyclohexane/ethylacetate 2:1) to yield the title compound.
LCMS (II) rt 2.71, m/z 263 (M+Na+CH₃CN)⁺.

### Step 2

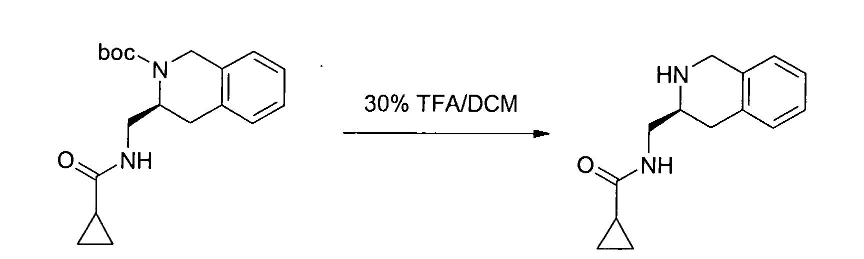

### (3S)-Cyclopropanecarboxylic acid (1,2,3,4-tetrahydro-isoquinolin-3-ylmethyl)-amide, trifluoroacetate salt

25 mg (0.08 mmol) of the product from step 1 are dissolved in 1 ml of dichloromethane and 0.5 ml of trifluoroacetic acid are added. The solution is stirred for 30 min at ambient temperature, then the solvent is removed under reduced pressure. The crude material is taken directly to the next step.
LCMS (II) rt 1.66, m/z 231 (M+H)⁺.

### Step 3

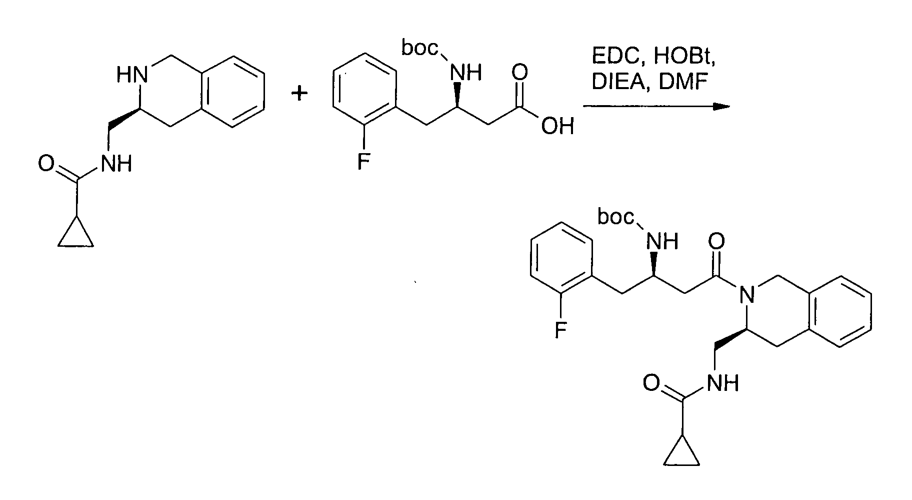

### [(3R)-3-((3S)-3-Cyclopropanecarbonyl-amino)-methyl)-3,4-dihydro-11H-isoquinoline-2-yl)-1-(2-fluoro-benzyl)-3-oxo-propyl]-carbamic acid tert-butyl ester

To a solution of 24 mg (0.08 mmol) Boc-(R)-3-amino-4-(2-fluoro-phenyl)-butyric acid in 2 ml N,N-dimethylformamide is added 19 mg (0.10 mmol) of 1-ethyl-3-(3-dimethylaminopropyl)-carbodiimide hydrochloride (EDC), 15 mg (0.10 mmol) of 1-hydroxybenzotriazole (HOBt) and 38 µl (0.20 mmol) diisopropylethylamine (DIEA) and after 5 min the crude material from step 2 (0.08 mmol) dissolved in 1 ml N,N-dimethylformamide. The mixture is stirred overnight at ambient temperature and diluted with ethyl acetate. The organic phase is washed sequentially with 5 % citric acid, saturated aqueous sodium bicarbonate solution, and brine, dried over sodium sulfate and concentrated in vacuo. Purification by flash chromatography (silica gel, cyclohexane/ethylacetate 2:1) afforded the title compound.
LCMS (II) rt 2.99, m/z 532 (M+Na)⁺.

### Step 4

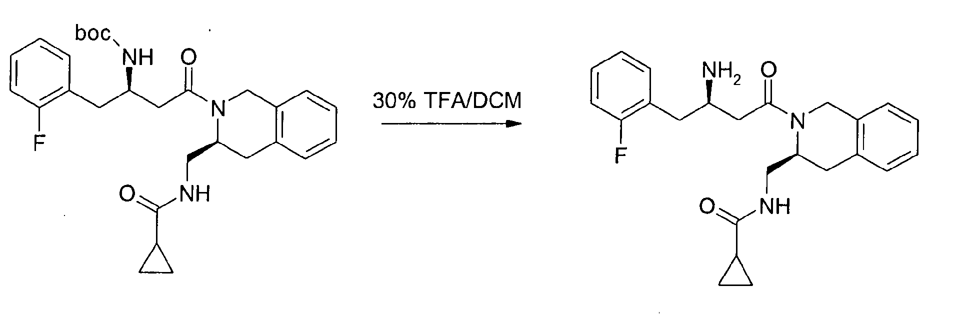

### Cyclopropanecarboxylic acid {2-[(3R)-3-amino-4-(2-fluoro-phenyl)-butyryl]-1,2,3,4-tetrahydro-isoquinolin-(3S)-3-ylmethyl}-amide, trifluoroacetate salt

26 mg (0.05 mmol) of the product from step 3 are dissolved in 2 ml of dichloromethane and 1 ml of trifluoroacetic acid are added. The solution is stirred for 30 min at ambient temperature and the solvents removed under reduced pressure. Purification by preparative HPLC gave the title compound.
LCMS (II) rt 2.21, m/z 410 (M+H)⁺.
¹H-NMR (300 MHz, DMSO-d₆) δ = 0.43.- 0.72 (m, 4H), 1.35 - 1.58 (m, 1H), 2.55 - 3.18 (m, 8H), 3.74 (m, 1H), 4.15 - 4.95 (m, 3H together with water), 7.17 - 7.39 (m, 8H), 7.90 (m, 3H), 8.18 (m, 1H).

### Example 3

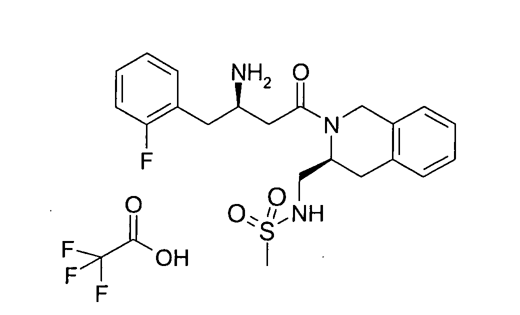

### Step 1

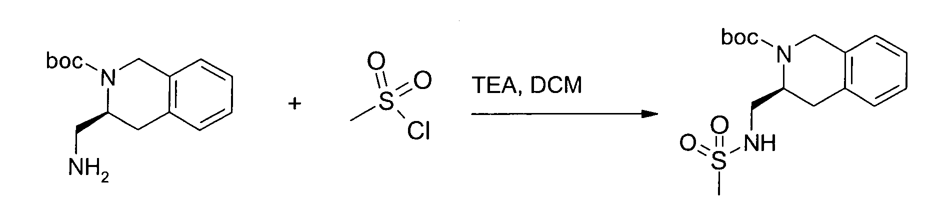

### (3S)-3-(Methanesulfonylamine-methyl)-3,4-dihydro-1H-isoquinoline-2-carboxylic acid tert-butyl ester

To a solution of 30 mg (0.11 mmol) of (3S)-3-Aminomethyl-3,4-dihydro-1H-isoquinoline-2-carboxylic acid tert-butyl ester in dichloromethane are added at 0°C 10 µl (0.13 mmol) of methanesulfonyl chloride and 18 µl (0.14 mmol) of triethylamine. The mixture is stirred for 3 h, then diluted with dichloromethane and washed with 5% citric acid solution, saturated sodium bicarbonate solution and brine and dried over sodium sulfate. Removal of the solvent in vacuo afforded the title compound that was used for the next step without further purification.
LCMS (II) rt 2.82, m/z 404 (M+Na+CH₃CN)⁺.

Following the procedures for step 2-4 from example 2 the title compound was prepared.

### N- {2-[(3R)-3-Amino-4-[2- fluoro-phenyl)-butyryl]-1,2,3,4-tetrahydro-isoquinolin-(3S)-3-ylmethyl}-methanesulfonamide, trifluoroacetate salt

LCMS (II) rt 2.10, m/z 420 (M+H)⁺.
¹H-NMR (300 MHz, DMSO-d₆) δ = 2.66 - 3.10 (m, 8H), 2.81 (s, 1.5H), 2.85 (s, 1.5H), 3.74 (m, 1H), 4.13 (d, J = 18.0 MHz, 0.5H), 4.16 (m, 0.5H), 4.37 (d, J = 16.7 Mhz, 0.5 H), 4.60 (d, J = 16.7 MHz, 0.5 H), 4.79 (m, 0.5 H), 4.96 (d, J = 18.0 MHz, 0.5H), 7.07 - 7.36 ( m, 8H), 7.91 (m, 4H).

### Example 4

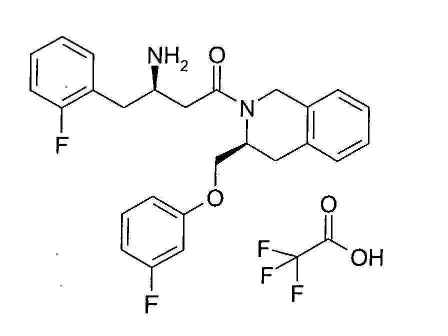

### Step 1

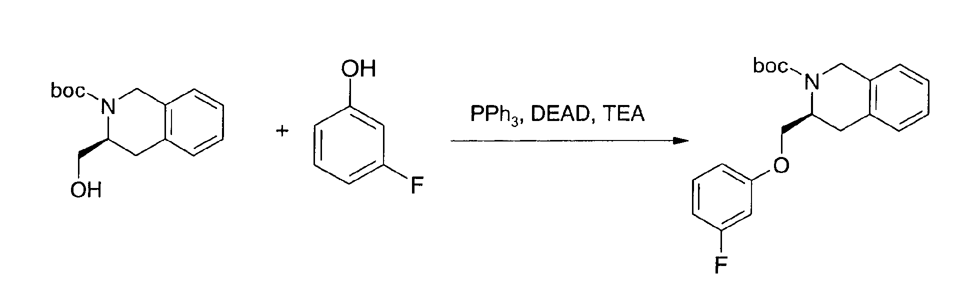

### (3S)-(3-Fluoro-phenoxymethyl)-3,4-dihydro-1H-isoquinoline-2-carboxylic acid tert-butyl ester

To a solution of 50 mg (0.19 mmol) of (3S)3-Hydroxymethyl-3,4-dihydro-1H-isoquinoline-2-carboxylic acid tert-butyl ester in 2 ml dry tetrahydrofuran is added at 0°C 57 mg ( 0.21 mmol) triphenylphosphine and 35 µl (0.23 mmol) diethyl azodicarboxylate. The mixture is stirred for 10 min before 19 mg (0.17 mmol) fluorophenol dissolved in 0.5 ml dry tetrahydrofurane are added. Stirring is continued at ambient temperature overnight. After evaporation of the solvent the remaining residue is purified by flash chromatography (silica gel, cyclohexane/ethylacetate = 2:1) to yield the title compound.
LCMS (II) rt 3.80, m/z 343 (M+H-CH₃)⁺.

Following the procedures for step 2-4 from example 2 the title compound was prepared.

### (3R)-3-Amino-1-[(3S)-3-(3-fluorophenoxyl)-3,4-dihydro-1H-isoquinolin-2-yl]-4-(2-fluorophenyl)-butan-1-one, trifluoroacetate salt

LCMS (II) rt 2.72, m/z 437 (M+H)⁺.
¹H-NMR (300 MHz, DMSO-d₆) δ = 2.74- 3.18 (m, 6H), 3.66 - 3.95 (m, 3H together with water), 4.13 (d, J = 17.6 MHz, 0.5H), 4.41 (d, J = 16.2 MHz, 0.5 H), 4.55 (m, 0.5H), 4.61 (d, J = 16.2 MHz, 0.5 H), 4.95 (m, 0.5 H), 4.96 (d, J = 17.6 MHz, 0.5H), 6.67 - 6.76 (m, 3H), 7.11 - 7.34 (m, 9H), 7.95 (m, 3H).

### Example 5

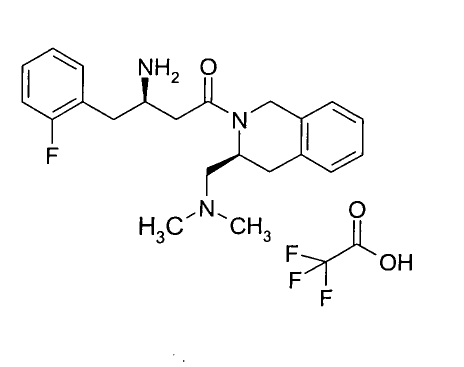

### Step 1

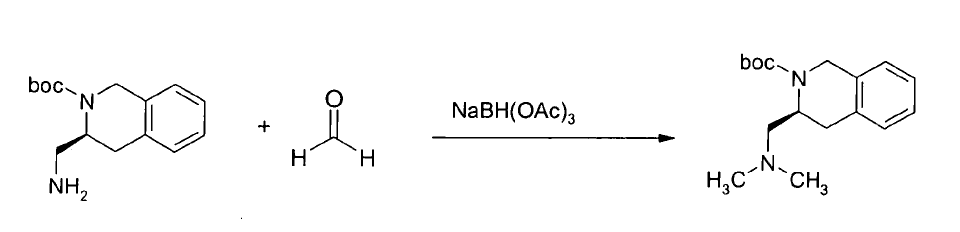

### (3S)-3-Dimethylaminomethyl-3,4-dihydro-1H-isoquinoline-2-carboxylic acid tert-butyl ester

To a solution of 20 mg (0.08 mmol) of (3S)-3-Aminomethyl-3,4-dihydro-1H-isoquinoline-2-carboxylic acid tert-butyl ester in 3 ml dichloroethane is added 17 ml (0.23 mmol) aqueous formaldehyde solution and 49 mg (0.23 mmol) of sodium triacetoxyborohydride. The mixture is stirred for 2 days at ambient temperature before saturated sodium bicarbonate solution is added. After extraction with ethylacetate and evaporation of the solvent the title compound was obtained.
LCMS (II) rt 2.17, m/z 291 (M+H)⁺.

Following the procedures for step 2-4 from example 2 the title compound was prepared.

### (3R)-3-Amino-1 -(3S)-3 -(3-dimethylaminomethyl-3,4-dihydro-1H-isoquinolin-2-yl)-4-(2-fluorophenyl)-butan-1-one, trifluoroacetate salt

LCMS (I) rt 3.42, m/z 370 (M+H)⁺.
¹H-NMR (300 MHz, DMSO-d₆) δ = 2.64 - 3.30 (m, 14H), 3.71 (m,1H), 4.45 (d, J = 16.8 Mhz, 1H), 4.72 (d, J = 16.8 MHz, 1H), 5.22 (m, 1 H), 7.12 - 7.38 (m, 8H), 7.95 (m, 3H), 9.40 (bs, 0.5H).

### Example 6

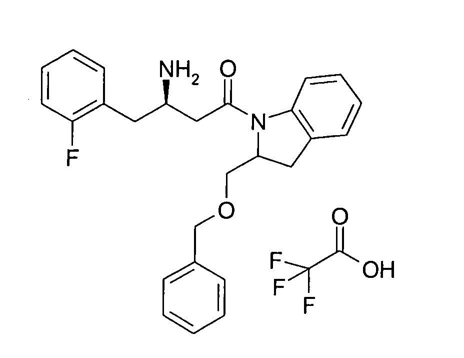

### Step 1

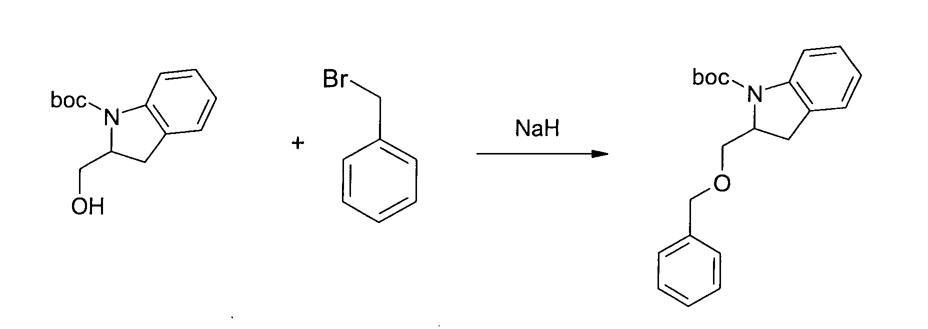

### 2-Benzyloxymethyl-2,3-dihydro-indole-1-carboxylic acid tert-butyl ester

To a solution of 30 mg (0.12 mmol) of 2-Hydroxymethyl-2,3-dihydro-indole-1-carboxylic acid tert-butyl ester in 2 ml dry tetrahydrofuran is added at 0°C 6 mg (0.13 mmol) sodium hydride and 17 µl (0.14 mmol) benzylbromide. The mixture is stirred at ambient temperature overnight before 5% citric acid solution and ethylacetate are added. The layers are separated, the organic layer washed subsequently with saturated sodium bicarbonate solution and brine and dried over sodium sulfate. After evaporation of the solvent the remaining residue is purified by flash chromatography (silica gel, cyclohexane/ethylacetate = 9:1) to yield the title compound.
LCMS (IV) rt 3.82, m/z 281 (M+H+CH₃CN-boc)⁺.

Following the procedures for step 2-4 from example 2 the title compound was prepared as a mixture of diastereomers.

### (3R)-3-Amino-1-benzyloxymethyl-2,3-dihydro-indo-1-yl)-4-(2-fluorophenyl)-butan-1-one, trifluoroacetate salt

LCMS (IV) rt 2.95, m/z 419 (M+H)⁺.

Using similar procedures as outlined above following examples were prepared.

### Example 7

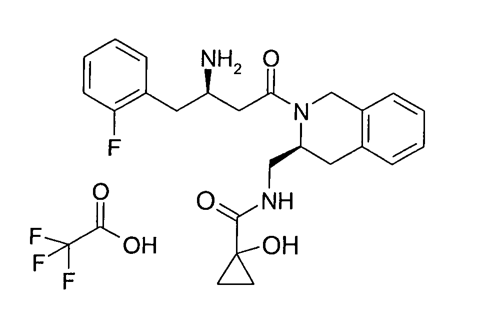

### 1-Hydroxy-Cyclopropanecarboxylic acid {2-[(3R)-3-amino-4-(2-fluoro-phenyl)-butyryl]-1,2,3,4-tetrahydro-isoquinolin-(3S)-3-ylmethyl}-amide, trifluoroacetate salt

LCMS (II) rt 205, m/z 426 (M+H)⁺.
¹H-NMR (300 MHz, DMSO-d₆) δ = 0.65 - 1.08 (m, 4H), 2.55 - 3.22 (m, 8H ), 3.74 (m, 1H), 4.15 - 4.95 (m, 3H together with water), 7.17 - 7.39 (m, 8H), 7.75 (m, 0.5H), 7.90 (m, 3H), 8.15 (m, 0.5H).

### Example 8

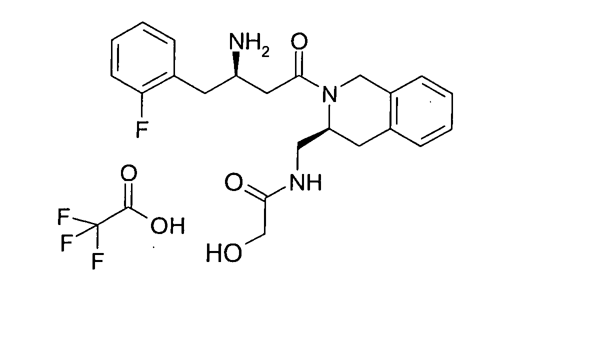

### N- {2-[(3R)-3-Amino-4-(2-fluoro-phenyl)-butyryl- 1,2,3,4-tetrahydroisoquinolin-(3S)-3-ylmethyl}-2-hydroxy-acetamide, trifluoroacetate salt

LCMS (I) rt 3.22, m/z 400 (M+H)⁺.
¹H-NMR (300 MHz, DMSO-d₆) δ = 2.61 - 3.17 (m, 8H), 3.60 - 4.20 (4H covered by water signal), 4.20 (d, J = 18.2 MHz, 0.7H), 4.29 (m, 0.7H), 4.47 (d, J = 16.5 Mhz, 0.3 H), 4.57 (d, J = 16.1 MHz, 0.3 H), 4.89 (m, 0.3 H), 4.96 (d, J = 17.6 MHz, 0.7H), 7.08 - 7.35 (m, 8H), 7.62 (m, 0.3H), 7.88 (m, 3H), 7.05 (m, 0.7H).

### Example 9

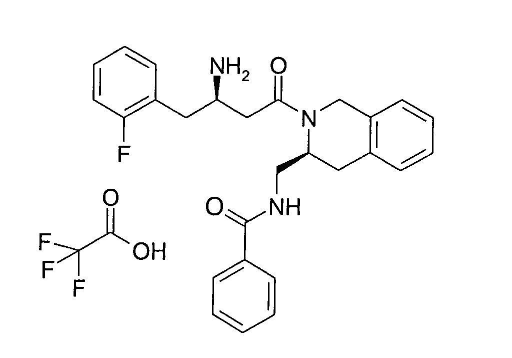

### N- {2-[(3R)-3-Amino-4-(2- fluoro-phenyl)-butyryl]-1 ,2,3,4-tetrahydro-isoquinolin-(3S)-3-ylmethyl}-benzamide, trifluoroacetate salt

LCMS (I) rt 4.67, m/z 446 (M+H)⁺.
¹H-NMR (300 MHz, DMSO-d₆) δ = 2.62 - 3.35 (m, 8H), 3.75 (m, 1H), 4.27 (d, J = 17.6 MHz, 0.5H), 4.41 (m, 0.5H), 4.56 (d, J = 16.0 MHz, 0.5H), 4.64 (d, J = 16.0 MHz, 0.5 H), 4.96 (d, J = 17.6 MHz, 0.5H), 5.07 (m, 0.5H), 7.03 - 7.82 (m, 13H), 7.85 - 7.98 (m, 3H), 8.35 (m, 0.5H), 8.60 (m, 0.5H).

### Example 10

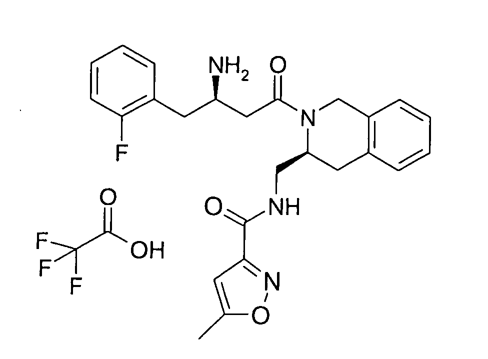

### 5-Methyl-isoxazole-3-carboxylic acid-{2-[(3R)-3-Amino-4-(2-fluoro-phenyl)-butyryl]-1 2,3,4-tetrahydro-isoquinolin-(3,S)-3-ylmethyl}-amide trifluoroacetate salt

LCMS (II) rt 2.28, m/z 451 (M+H)⁺.
¹H-NMR (300 MHz, DMSO-d₆) δ = 2.32 (s, 1.5H), 2.45 (s, 1.5H), 2.57 - 3.28 (m, 8H), 3.63 - 3.71 (m, 1H), 4.27 (d, J = 17.6 MHz, 0.5H), 4.41 (m, 0.5H), 4.56 (d, J = 16.0 Mhz, 0.5 H), 4.64 (d, J = 16.0 MHz, 0.5 H), 4.96 (d, J = 17.6 MHz, 0.5H), 5.07 (m, 0.5 H), 6.30 (s, 0.5H), 6.49 (s, 0.5H), 7.05 - 7.38 (m, 8H), 7.85 - 7.98 (m, 3H), 8.34 (m, 0.5H), 8.81 (m, 0.5H).

### Example 11

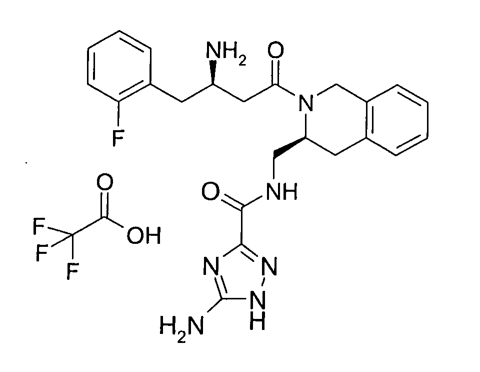

### 5-Amino-1H-[1.2,4]triazole-3-carboxylic acid- {2-[(3R)-3-Amino-4-(2- fluoro-phenyl)-butyryl]- 1 ,2,3 ,4-tetrahydro-isoquinolin-(3S)-3-ylmethyl}-amide, trifluoroacetate salt

LCMS (I) rt 2.84, m/z 452 (M+H)⁺.

### Example 12

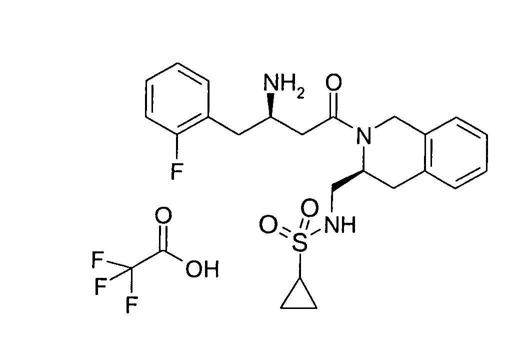

### Cyclopropanesulfonic acid {2-[(3R)-3-amino-4-(2- fluoro-phenyl)-butyryl] -1,2,3,4-tetrahydro-isoquinolin-(3S)-3-ylmethyl}-amide, trifluoroacetate salt

LCMS (II) rt 2.30, m/z 446 (M+H)⁺.
¹H-NMR (300 MHz, DMSO-d₆) δ = 0.81 - 0.93 (m, 4H), 2.45 (m, 1H), 2.66 - 3.10 (m, 8H ), 3.74 (m, 1H), 4.13 (d, J = 18.0 MHz, 0.5H), 4.16 (m, 0.5H), 4.37 (d, J = 16.7 Mhz, 0.5 H), 4.60 (d, J = 16.7 MHz, 0.5 H), 4.79 (m, 0.5 H), 4.96 (d, J = 18.0 MHz, 0.5H), 7.07 - 7.36 ( m, 8H), 7.91 (m, 4H):

### Example 13

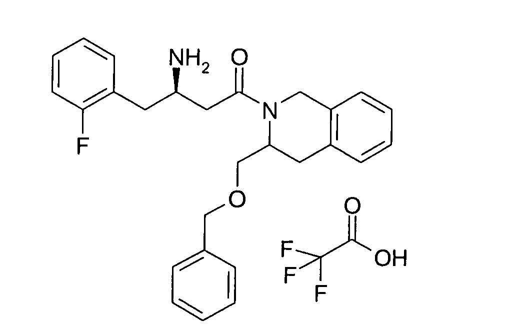

### (3R)-3-Amino-1-benzyloxmethyl-3,4-dihydro-1H-isoquinolin-2-yl)-4-(2-fluorophenyl)-butan-1-one, trifluoroacetate salt

LCMS (II) rt 2.59, m/z 433 (M+H)⁺.

### ASSAYS

Inhibition of DPP-IV peptidase activity was monitored with a continuous fluorimetric assay. This assay is based on the cleavage of the substrate Gly-Pro-AMC (Bachem) by DPP-IV, releasing free AMC. The assay is carried out in 96-well microtiterplates. In a total volume of 100 µl, compounds are preincubated with 50 pM DPP-IV employing a buffer containing 10mM Hepes, 150mM NaCl, 0.005% Tween 20 (pH 7.4). The reaction is started by the addition of 16 µM substrate and the fluorescence of liberated AMC is detected for 10 minutes at 25 °C with a fluorescence reader (BMG-Fluostar; BMG-Technologies) using an excitation wavelength of 370 nm and an emission wavelength of 450 nm. The final concentration of DMSO is 1 %. The inhibitory potential of the compounds were determined. DPP-IV activity assays were carried out with human and porcine DPP-IV (see below); both enzymes showed comparable activities.

Soluble human DPP-IV lacking the transmembrane anchor (Gly31-Pro766) was expressed in a recombinant YEAST-strain as Pre-Pro-alpha-mating fusion. The secreted product (rhuDPP-IV-Gly31-Pro766) was purified from fermentation broth (>90% purity).

The examples 2-13 show a % inhibition of about 2 µM or less.

## Claims

1. A compound of formula (I) 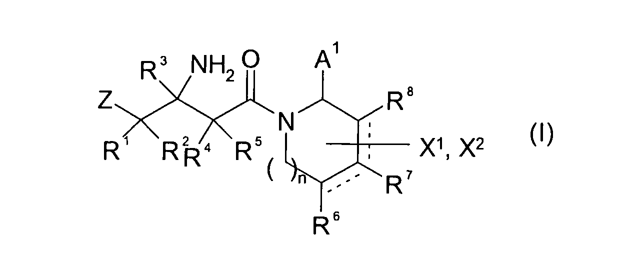 or a pharmaceutically acceptable salt thereof, wherein a dotted line indicates an optionally present double bond and wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R⁹, wherein R⁹ is independently selected from the group consisting of halogen; CN; OH; NH₂; oxo (=O), where the ring is at least partially saturated; R¹⁰; and R¹¹;
R¹⁰ is selected from the group consisting of C₁₋₆ alkyl; O-C₁₋₆ alkyl; and S-C₁₋₆ alkyl, wherein R¹⁰ is optionally interrupted by oxygen and wherein R¹⁰ is optionally substituted with one or more halogen independently selected from the group consisting of F; and Cl;
R¹¹ is selected from the group consisting of phenyl; heterocycle; and C₃₋₇ cycloalkyl, wherein R¹¹ is optionally substituted with one or more R¹², wherein R¹² is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R^{4a};
R², R⁵ are independently selected from the group consisting of H; F; and R^{4b};
R^{4a} is independently selected from the group consisting of C₁₋₆ alkyl; and O-C₁₋₆ alkyl, wherein R^{4a} is optionally substituted with one or more halogen independently selected from the group consisting of F; and Cl;
R^{4b} is C₁₋₆ alkyl, wherein R^{4b} is optionally substituted with one or more halogen independently selected from the group consisting of F; and Cl;
R³ is selected from the group consisting of H; and C₁₋₆ alkyl;
Optionally one or more pairs of R¹, R², R³, R⁴, R⁵ independently selected from the group consisting of R¹/R²; R²/R³ ; R³/R⁴; and R⁴/R⁵, form a C₃₋₇ cycloalkyl ring, which is optionally substituted with one or more of R¹³, wherein R¹³ is independently selected from the group consisting of F; Cl; and OH;
n is 0, 1 or 2;
X¹ is selected from the group consisting of H; F; Cl; OH; and C₁₋₆ alkyl, optionally substituted with one or more halogen selected from the group consisting of F; and Cl;
X² is selected from the group consisting of H; F; Cl; and C₁₋₆ alkyl, optionally substituted with one or more halogen selected from the group consisting of F; and Cl;
R⁶ and R⁷ form together a ring A, provided that R⁸ is selected from the group consisting of H; F; Cl; Y-H; Y-T; Y¹-H; Y¹-T; and C₁₋₆ alkyl, optionally substituted with one or more halogen selected from the group consisting of F; and Cl;
or
R⁸ and R⁷ form together a ring A, provided that R⁶ is selected from the group consisting of H; F; Cl; OH; and C₁₋₆ alkyl, optionally substituted with one or more halogen selected from the group consisting of F; and Cl;
A is selected from the group consisting of Z¹; and Z²;
Z¹ is selected from the group consisting of phenyl; naphthyl; and indenyl; wherein Z¹ is optionally substituted with one or more R¹⁴; wherein R¹⁴ is independently selected from the group consisting of halogen; CN; C₁₋₆ alkyl; COOR¹⁵; OR¹⁵; C(O)N(R¹⁵)₂; S(O)₂N(R¹⁵)₂; S(O)N(R¹⁵)₂, N(R¹⁵)S(O)₂R¹⁵; and N(R¹⁵)S(O)R¹⁵;
Z² is selected from the group consisting of C₃₋₇ cycloalkyl; indanyl; tetralinyl; decalinyl; heterocycle; and heterobicycle; wherein Z² is optionally substituted with one or more R¹⁶, wherein R¹⁶ is independently selected from the group consisting of halogen; CN; C₁₋₆ alkyl; OR¹⁷; oxo (=O), where the ring is at least partially saturated; N(R¹⁷)₂; COOR¹⁷; C(O)N(R¹⁷)₂; S(O)₂N(R¹⁷)₂; S(O)N(R¹⁷)₂; N(R¹⁷)S(O)₂R¹⁷ ; and N(R¹⁷)S(O)R¹⁷;
R¹⁵ , R¹⁷ are independently selected from the group consisting of H; C₃₋₇ cycloalkyl; C₁₋₆ alkyl; and -C₁₋₆ alkyl-C₃₋₇ cycloalkyl; wherein each C₃₋₇ cycloalkyl and C₁₋₆ alkyl is optionally substituted with one or more F;
A¹ is selected from the group consisting of F; Cl; and Y-T; provided that when A¹ is Y-T, R⁸ is other than Y-T or Y¹-T;
Optionally A¹ is selected from the group consisting of H; CN; and Y-H, provided that n is other than 1 or R⁸ is selected other than to form a ring A; or R⁸ and R⁷ form a ring A other than pyrimidine;
Y is selected from the group consisting of -C₁₋₆ alkyl-O-T⁰-; -C₁₋₆ alkyl-N(R¹⁸)-T⁰-; -C₁₋₆ alkyl-S-T⁰-; -C₁₋₆ alkyl-S(O)-T⁰-; and -C₁₋₆ alkyl-S(O)₂-T⁰-; wherein each C₁₋₆ alkyl is optionally substituted with one or more F;
Y¹ is selected from the group consisting of -O-T⁰-; -N(R^{18a})-T⁰-; -S-T⁰-; -S(O)-T⁰-; and -S(O)₂-T⁰-;
R¹⁸, R^{18a} are independently selected from the group consisting of H; T⁰-H; and T⁰-T;
T⁰ is selected from the group consisting of a covalent bond; -C₁₋₆ alkyl-; -C₁₋₆ alkyl-O-; -C₁₋₆ alkyl-N(R¹⁹)-; -C(O)-; -C(O)-C₁₋₆ alkyl-; -C(O)-C₁₋₆ alkyl-O-; -C(O)-C₁₋₆ alkyl-N(R¹⁹)-; -C(O)O-; -C(O)O-C₁₋₆ alkyl-; -C(O)O-C₁₋₆ alkyl-O-; -C(O)O-C₁₋₆ alkyl-N(R¹⁹)-; -C(O)N(R¹⁹)-; -C(O)N(R¹⁹)-C₁₋₆ alkyl-; -C(O)N(R¹⁹)-C₁₋₆ alkyl-O-; - C(O)N(R¹⁹)-C₁₋₆ alkyl-N(R²⁰)-; -S(O)₂-; -S(O)₂-C₁₋₆ alkyl-;
-S(O)₂-C₁₋₆ alkyl-O-; and -S(O)₂-C₁₋₆ alkyl-N(R¹⁹)-; wherein each C₁₋₆ alkyl is optionally substituted with one or more F;
R¹⁹, R²⁰ are independently selected from the group consisting of H; and C₁₋₆ alkyl;
T is selected from the group consisting of T¹; and T²;
T¹ is selected from the group consisting of phenyl; naphthyl; and indenyl; wherein T¹ is optionally substituted with one or more R²¹; wherein R²¹ is independently selected from the group consisting of halogen; CN; R²²; COOH; OH; C(O)NH₂; S(O)₂NH₂; S(O)NH₂; COOT³; OT³; ST³; C(O)N(R²³)T³; S(O)₂N(R²³)T³_{;} S(O)N(R²³)T³ and T³;
T² is selected from the group consisting of C₃₋₇ cycloalkyl; indanyl; tetralinyl; decalinyl; heterocycle; and heterobicycle; wherein T² is optionally substituted with one or more R²⁴, wherein R²⁴ is independently selected from the group consisting of halogen; CN; R²⁵; OH; oxo (=O), where the ring is at least partially saturated; NH₂; COOH; C(O)NH₂; S(O)₂NH₂; S(O)NH₂; COOT³; OT³; C(O)N(R²⁶)T³; S(O)₂N(R²⁶)T³; S(O)N(R²⁶)T³; N(R²⁶)T³; and T³;
R²² is selected from the group consisting of C₁₋₆ alkyl; O-C₁₋₆ alkyl; S-C₁₋₆ alkyl; COO-C₁₋₆ alkyl; OC(O)- C₁₋₆ alkyl; C(O)N(R²⁷)-C₁₋₆ alkyl; S(O)₂N(R²⁷)-C₁₋₆ alkyl; S(O)N(R²⁷)-C₁₋₆alkyl; S(O)-C₁₋₆ alkyl; S(O)₂-C₁₋₆ alkyl; N(R²⁷)S(O)₂-C₁₋₆ alkyl; and N(R²⁷)S(O) -C₁₋₆ alkyl; wherein each C₁₋₆ alkyl is optionally substituted with one more R²⁸, wherein R²⁸ is independently selected from the group consisting of F; COOR²⁹; C(O)N(R²⁹R³⁰); S(O)₂N(R²¹R³⁰); OR²⁹; N(R²⁹R³⁰); T³; O-T³; and N(R²⁹)-T3;
R²⁵ is selected from the group consisting of C₁₋₆ alkyl; O-C₁₋₆ alkyl; S-C₁₋₆ alkyl; N(R³¹)-C₁₋₆ alkyl; COO-C₁₋₆ alkyl; OC(O)- C₁₋₆ alkyl; C(O)N(R³¹)- C₁₋₆ alkyl; N(R³¹)-C(O)-C₁₋₆ alkyl; S(O)₂N(R³¹)-C₁₋₆ alkyl; S(O)N(R³¹)-C₁₋₆ alkyl; S(O)-C₁₋₆ alkyl; S(O)₂-C₁₋₆ alkyl; -N(R³¹)S(O)₂-C₁₋₆ alkyl; and -N(R³¹)S(O)-C₁₋₆ alkyl; wherein each C₁₋₆ alkyl is optionally substituted with one or more R³², wherein R³² is independently selected from the group consisting of F; COOR³³; C(O)N(R³³R³⁴); S(O)₂N(R³³R³⁴); S(O)N(R³³R³⁴); OR³³; N(R³³R³⁴); T³; O-T³; and N(R³³)-T³;
R^{23,} R²⁶, R²⁷, R²⁹, R³⁰, R³¹, R³³, R³⁴ are independently selected from the group consisting of H; and C₁₋₆ alkyl;
T³ is selected from the group consisting of T⁴; and T⁵;
T⁴ is selected from the group consisting of phenyl; naphthyl; and indenyl; wherein T⁴ is optionally substituted with one or more R³⁵, wherein R³⁵ is independently selected from the group consisting of halogen; CN; COOR³⁶; OR³⁶; C(O)N(R³⁶R³⁷); S(O)₂N(R³⁶R³⁷); C₁₋₆ alkyl; O-C₁₋₆ alkyl; S-C₁₋₆ alkyl; COO-C₁₋₆ alkyl; OC(O)-C₁₋₆ alkyl; C(O)N(R³⁶)- C₁₋₆ alkyl; S(O)₂N(R³⁶)-C₁₋₆ alkyl; S(O)N(R³⁶)-C₁₋₆ alkyl; S(O)₂-C₁₋₆ alkyl; S(O) -C₁₋₆ alkyl; N(R³⁶)S(O)₂-C₁₋₆ alkyl; and N(R³⁶)S(O)-C₁₋₆ alkyl; wherein each C₁₋₆ alkyl is optionally substituted with one more halogen selected from the group consisting of F; and Cl;
T⁵ is selected from the group consisting of heterocycle; heterobicycle; C₃₋₇ cycloalkyl; indanyl; tetralinyl; and decalinyl; wherein T⁵ is optionally substituted with one or more R³⁸, wherein R³⁸ is independently selected from the group consisting of halogen; CN; OR³⁹; oxo (=O), where the ring is at least partially saturated; N(R³⁹R⁴⁰); COOR³⁹; C(O)N(R³⁹R⁴⁰); S(O)₂N(R³⁹R⁴⁰); S(O)N(R³⁹R⁴⁰); C₁₋₆ alkyl; O-C₁₋₆ alkyl; S-C₁₋₆ alkyl; N(R³⁹)-C₁₋₆ alkyl; COO-C₁₋₆ alkyl; OC(O)- C₁₋ ₆ alkyl; C(O)N(R³⁹)- C₁₋₆ alkyl; N(R³⁹)-C(O)-C₁₋₆ alkyl; S(O)₂N(R³⁹)-C₁₋₆ alkyl; S(O)N(R³⁹)-C₁₋₆ alkyl; S(O)₂-C₁₋₆ alkyl; S(O)-C₁₋₆ alkyl; N(R³⁹)S(O)₂-C₁₋₆ alkyl; and N(R³⁹)S(O)-C₁₋₆ alkyl; wherein each C₁₋₆ alkyl is optionally substituted with one more halogen selected from the group consisting of F; and Cl;
R³⁶, R³⁷, R³⁹, R⁴⁰, are independently selected from the group consisting of H; and C₁₋₆alkyl.

2. A compound according to claim 1 of formula (Ia) 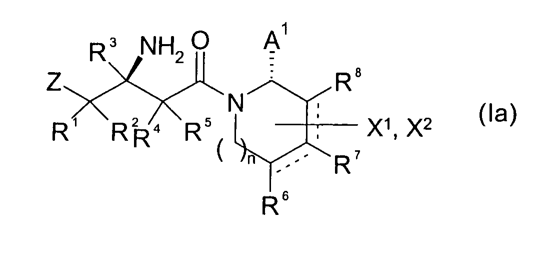 or a pharmaceutically acceptable salt thereof, wherein Z, R¹⁻⁸, n, A¹, X¹ and X² have the meaning as indicated in claim 1.

3. A compound according to claim 1 or 2, wherein Z is selected from the group consisting of phenyl; and heterocycle; and wherein Z is optionally substituted with up to 2 R⁹, which are the same or different.

4. A compound according to any one of the preceding claims, wherein R⁹ is selected from the group consisting of F; Cl; CN; and C₁₋₆ alkyl.

5. A compound according to any one of the preceding claims, wherein R¹, R², R⁴, R⁵ are independently selected from the group consisting of H; F; and C₁₋₆ alkyl, optionally substituted with one or more F.

6. A compound according to any one of the preceding claims, wherein R³ is H.

7. A compound according to any one of the preceding claims, wherein n is 0 or 1.

8. A compound according to any one of the preceding claims, wherein X¹, X² are independently selected from the group consisting of H; and F.

9. A compound according to any one of the preceding claims, wherein R⁶ and R⁷ form together a ring A and R⁸ is selected from the group consisting of H; and F.

10. A compound according to any one of the preceding claims, wherein A is selected from the group consisting of phenyl, optionally substituted with up to 3 R¹⁴, which are the same or different; and C₃₋₇ cycloalkyl, optionally substituted with up to 3 R¹⁶, which are the same or different.

11. A compound according to any one of the preceding claims, wherein R¹⁴, R¹⁶ are independently selected from the group consisting of halogen; CN; and CH₃.

12. A compound according to any one of the preceding claims, wherein A¹ is selected from the group consisting of H; and Y-H and R⁸ is selected other than to form a ring A;

13. A compound according to claim 12, wherein Y is selected from the group consisting of C₁₋₆ alkyl-N(R¹⁸); C₁₋₆ alkyl-N(R¹⁸)-C₁₋₆ alkyl; C₁₋₆ alkyl-N(R¹⁸)-C(O)-C₁₋₆ alkyl; C₁₋₆ alkyl-N(R¹⁸)-C(O)-C₁₋₆ alkyl-O; C₁₋₆ alkyl-N(R¹⁸)-S(O)₂-C₁₋₆ alkyl; and C₁₋₆ alkyl-O, wherein R¹⁸ is selected from the group consisting of H; and C₁₋₆ alkyl.

14. A compound according to claim 13, wherein Y is selected from the group consisting of -CH₂-N(R¹⁸); -CH₂-N(R¹⁸)-CH₂-; -CH₂-N(R¹⁸)-C(O)-CH₂-; -CH₂-N(R¹⁸)-S(O)₂-CH₂-; -CH₂-N(R¹⁸)-C(O)-CH₂-O-; and -CH₂-O-; wherein R¹⁸ is selected from the group consisting of H; and CH₃.

15. A compound according to any one of claims 1 to 11, wherein A¹ is Y-T.

16. A compound according to claim 15, wherein Y is selected from the group consisting of C₁₋₆ alkyl-N(R¹⁸); C₁₋₆ alkyl-N(R¹⁸)-C₁₋₆ alkyl; C₁₋₆ alkyl-N(R¹⁸)-C(O); C₁₋₆ alkyl-N(R¹⁸)-C(O)-C₁₋₆ alkyl; C₁₋₆ alkyl-N(R¹⁸)-S(O)₂; C₁₋₆ alkyl-N(R¹⁸)-S(O)₂-C₁₋₆ alkyl; C₁₋₆ alkyl-O; and C₁₋₆ alkyl-O-C₁₋₆ alkyl; wherein R¹⁸ is selected from the group consisting of H; and C₁₋₆ alkyl.

17. A compound according to claim 16, wherein Y is selected from the group consisting of CH₂-NH-C(O); CH₂-O; CH₂-O-CH₂-; and CH₂-NHS(O)₂.

18. A compound according to any one of the preceding claims, wherein T is T¹ and T¹ is phenyl, optionally substituted with up to 3 R²¹ which are the same or different.

19. A compound according to claim 18 wherein R²¹ is F.

20. A compound according to any one of the preceding claims, wherein T is T² and T² is selected from the group consisting of C₃₋₇ cycloalkyl; and heterocycle, wherein T² is optionally substituted with up to 3 R²⁴, which are the same or different.

21. A compound according to claim 20, wherein T² is selected from the group consisting of cyclopropyl; 1,2,4-triazole; and 1,2-oxazole.

22. A compound according to claim 20, wherein R²⁴ is selected from the group consisting of OH; NH₂; and CH₃.

23. A compound according to claim 1 selected from the group consisting of

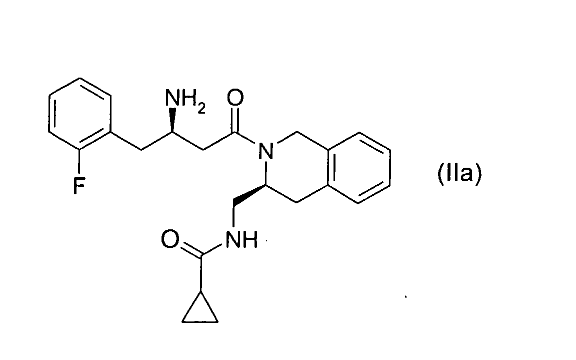

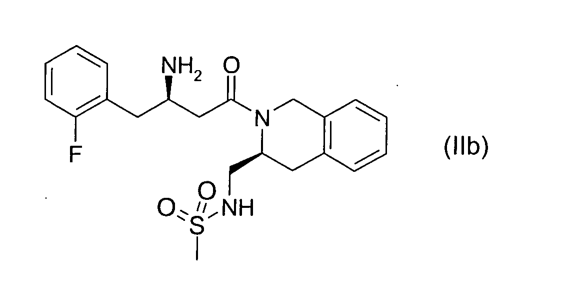

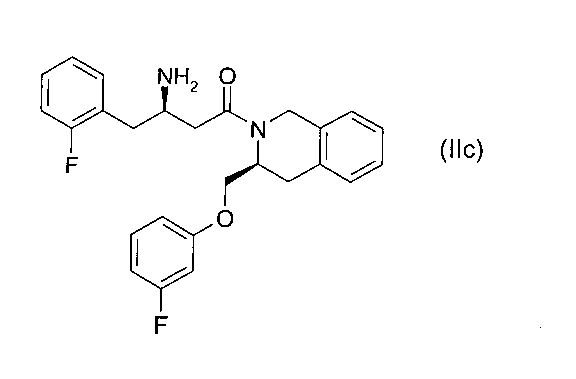

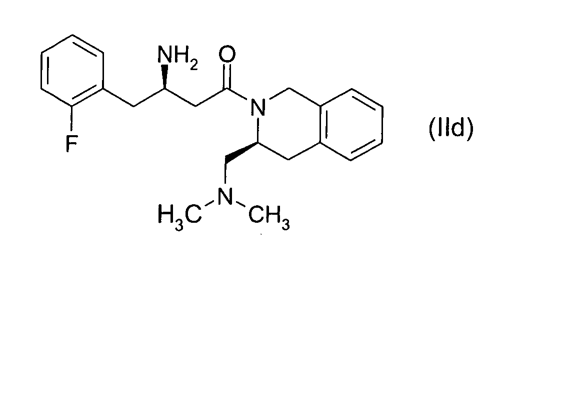

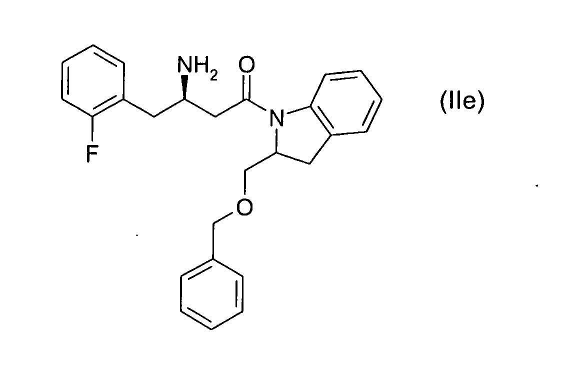

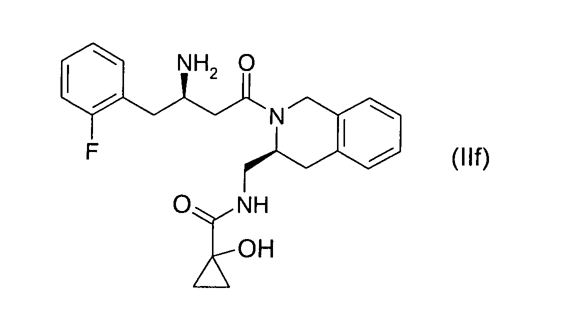

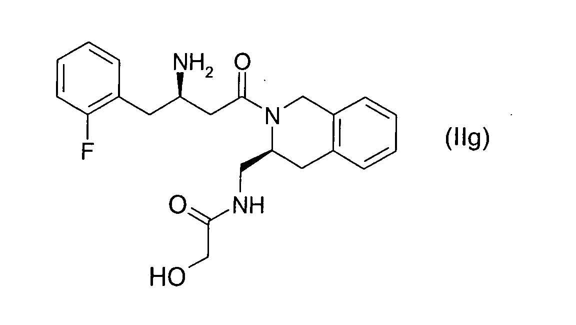

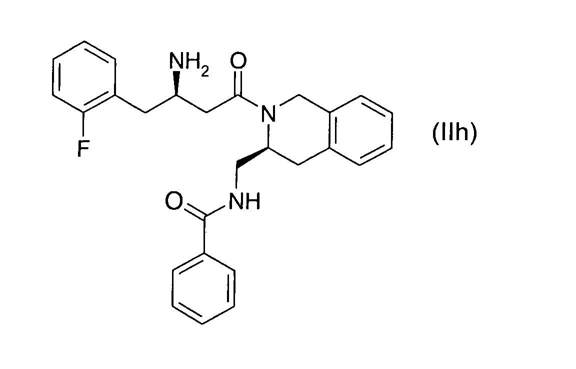

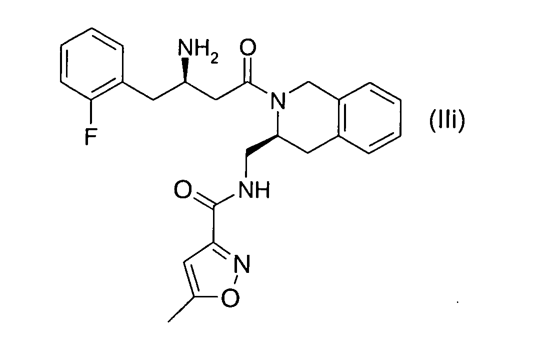

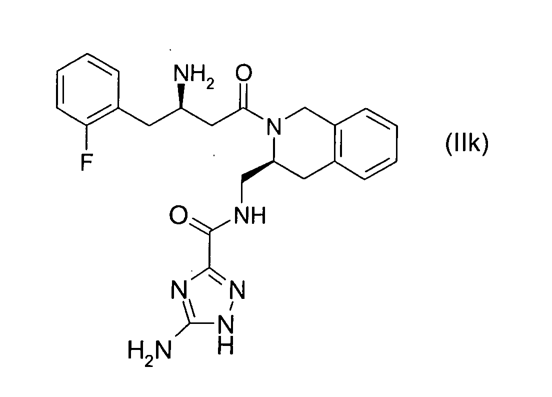

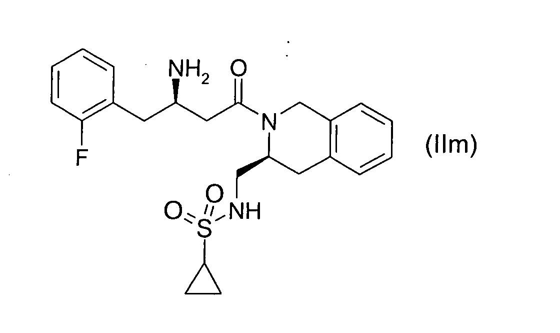

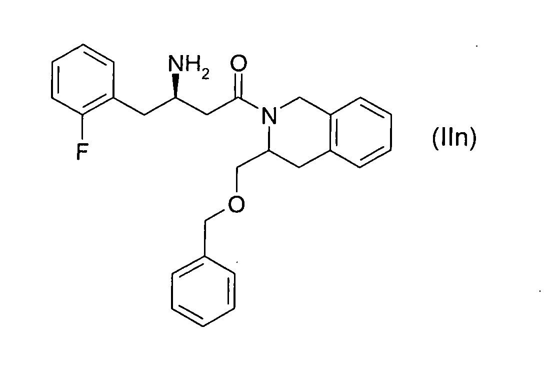

24. A prodrug compound of a compound according to any one of the claims 1 to 23.

25. A pharmaceutical composition comprising a compound or a pharmaceutically acceptable salt thereof according to any one of the claims 1 to 24 together with a pharmaceutically acceptable carrier.

26. A pharmaceutical composition according to claim 25, comprising one or more additional compounds or pharmaceutically acceptable salts thereof selected from the group consisting of another compound according to any one of the claims 1 to 24; another DPP-IV inhibitor; insulin sensitizers; PPAR agonists; biguanides; protein tyrosinephosphatase-1B (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoIy dual agonists; inhibitors of cholesterol absorption; acyl CoA : cholesterol acyltransferase inhibitors; anti-oxidants; PPARo agonists; antiobesity compounds; an ileal bile acid transporter inhibitor; and anti-inflammatory agents.

27. A compound or a pharmaceutically acceptable salt thereof of any one of the claims 1 to 24 for use as a medicament.

28. Use of a compound or a pharmaceutically acceptable salt thereof of any of the claims 1 to 24 for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency.

29. Use of a compound according to any one of the claims 1 to 24 as a DPP-IV inhibitor.
